# EUROPEAN PATENT SPECIFICATION

(11) **EP 4 479 367 B1**
(45) Date of publication and mention of the grant of the patent: **28.10.2026**
(21) Application number: 23705395.4
(22) Date of filing: 15.02.2023
(51) Int. Cl.: C07C 29/09

(54) **A METHOD FOR THE PRODUCTION OF ONE OR MORE BIOPOLYOLS, AND A COMPOSITION COMPRISING ONE OR MORE BIOPOLYOLS PRODUCED THEREFROM**
VERFAHREN ZUR HERSTELLUNG EINES ODER MEHRERER BIOPOLYOLE UND ZUSAMMENSETZUNG MIT EINEM ODER MEHREREN DARAUS HERGESTELLTEN BIOPOLYOLEN
PROCÉDÉ DE PRODUCTION D'UN OU DE PLUSIEURS BIOPOLYOLS ET COMPOSITION COMPRENANT UN OU PLUSIEURS BIOPOLYOLS AINSI PRODUITS

(30) Priority: 16.02.2022 GB 202202044
(43) Date of publication of application: 25.12.2024
(73) Proprietor: Ecorbio Ltd, Nicosia 2414 (CY)
(72) Inventor: JASIUNAS, Lukas, 2414 Nicosia (CY)
(74) Representative: Boult Wade Tennant LLP
(86) International application number: PCT/EP2023/053726
(87) International publication number: WO 2023/156438

(56) References cited:
- KR-B1- 102 245 442
- US-A1- 2012 165 494
- JASIUNAS LUKAS ET AL: "Wet and Coarse: The Robustness of Two-Stage Crude Glycerol Mediated Solvothermal Liquefaction of Residual Biomass", WASTE AND BIOMASS VALORIZATION, SPRINGER NETHERLANDS, NL, vol. 11, no. 5, 17 September 2018 (2018-09-17), pages 2171 - 2181, XP037082285, ISSN: 1877-2641, [retrieved on 20180917], DOI: 10.1007/S12649-018-0453-0
- OLSZEWSKI ADAM ET AL: "Synthesis and characterization of biopolyols through biomass liquefaction of wood shavings and their application in the preparation of polyurethane wood composites", HOLZ ALS ROH- UND WERKSTOFF, SPRINGER-VERLAG. BERLIN, DE, vol. 80, no. 1, 22 November 2021 (2021-11-22), pages 57 - 74, XP037688321, ISSN: 0018-3768, [retrieved on 20211122], DOI: 10.1007/S00107-021-01755-6

## Description

The present invention relates to a method for the production of one or more biopolyols from a feedstock, and in particular to the production of one or more biopolyols from a feedstock comprising waste biomass and a glycerol- or glyceride-containing composition.

### BACKGROUND OF THE INVENTION

Polyurethane is used across a wide range of areas, and has been a commonly used polymer since the 1950's. Products containing polyurethane are used across many industries, and are prevalent in everyday life. Examples of such common polyurethane-based products include thermally insulated construction elements, domestic use construction filler foams and commercial and domestic wood adhesives. These polyurethane-based articles are present in many end-user products such as building elements, furniture and automotive interiors.

Polyols are known to be a major constituent in polyurethane synthesis, and are conventionally produced using petroleum derivatives. Polyols are organic compounds having more than one hydroxyl group, and can include diols, triols, tetrols, and so on. Polyols form polyurethanes by the known reaction between an isocyanate and a polyol, as shown below.

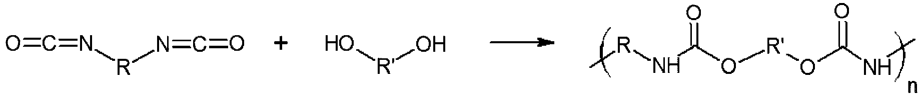

Biopolyols, which include natural oil polyols (NOPs) and sugar alcohols, are polyols derived from biological mass, such as vegetable oils or plant matter. The primary use of these materials is in the production of polyurethanes. A main source of the biopolyols is the triglycerides in vegetable oil, for example, castor oil.

Recent concerns regarding crude oil reserves and the environmental impact of fossil resource extraction have resulted in there being a need for polyols that are derived from alternative, more sustainable sources, whilst still being appropriate for use in polyurethane synthesis. In addition, large non-governmental organizations (NGOs), such as the United Nations (UN), are encouraging a shift towards using renewable resources and sustainable industrial processes via well-known initiatives such as the Sustainable Development Goals (SDGs).

Various methods of producing sustainable biopolyols have been investigated, and chemicals sourced from biomass feedstocks are generally on the rise. However, due to the immense size and demand of the polyol market (i.e. estimated to be worth USD 45 billion by 2025 at a compound annual growth rate of 8.5%), further sustainable methods of making biopolyols are required. In fact, out of the current global polyol market, biopolyols make up about 10 % already, a fraction that is increasing each year. There is a need for alternative sustainable, waste-derived biopolyols, and methods of making said waste-derived biopolyols, as the polyol market is still heavily dominated by fossil-derived polyols.

Known and presently used methods of making biopolyols typically utilize feedstocks competitive with food crops or ones of high indirect land use changes (ILUC). The currently used processes also suffer from high feedstock costs, and the majority of modern biorefineries still rely on neat solvents and extensive post-treatment steps. In addition, many known processes for making biopolyols, such as those described in EP 3689847, US 2020/0308500, CN 111499861, KR 20180002125, EP3138869 and US 8022257, have not proven able to take a low quality feedstock, and produce a high quality biopolyol product mixture, whilst maintaining a good conversion ratio.

"Wet and Coarse: The Robustness of Two-Stage Crude Glycerol Mediated Solvothermal Liquefaction of Residual Biomass" by Jasiunas et al, Waste and Biomass Valorization (2020) 11:2171-2181 https://doi.org/10.1007/s12649-018-0453-0, discloses a method for treating waste biomass. "Mechanical, thermal properties and stability of high renewable content liquefied residual biomass derived bio-polyurethane wood adhesives" by Jasiunas et al., (2020); International Journal of Adhesion and Adhesives. 101. 102618. 10.1016/j.ijadhadh.2020.102618, and "Mechanical, Thermal Properties and Stability of Rigid Polyurethane Foams Produced with Crude-Glycerol Derived Biomass Biopolyols" by Jasiunas, Lukas et al. (2020); Journal of Polymers and the Environment. 28. 10.1007/s10924-020-01686-y. relate to foams and adhesives made from biopolyols.

Accordingly, it is desirable to provide a method for the production of biopolyols that uses less expensive feedstocks, that can be obtained with minimal land usage, and which require minimum post-treatment processing or neat solvent, and/or that tackles at least some of the problems associated with the prior art or, at least, to provide a commercially viable alternative thereto. In particular, it is desirable to provide a method for the production of one or more biopolyols that can produce high quality products from low quality feedstock, whilst exhibiting acceptable conversion ratios. Olszewski, A., Kosmela, P. & Piszczyk, 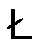 . Synthesis and characterization of biopolyols through biomass liquefaction of wood shavings and their application in the preparation of polyurethane wood composites. *Eur. J. Wood Prod.* **80,** 57-74 (2022) relates to the utilization of forestry and agricultural waste materials by synthesis of bio-based polyols and manufacturing of polyurethane wood composites. KR 102245442 B1 relates to a method for producing biopolyol from lignocellulosic biomass and a method for producing biopolyurethane. US 2012/165494 A1 relates to methods for producing polyols and polyurethanes.

### SUMMARY OF THE INVENTION

The invention is in accordance with the appended claims. According to a first aspect there is provided a method for the production of one or more biopolyols from a feedstock, the method comprising:
(i) providing a feedstock comprising waste biomass and a glycerol- or glyceride-containing composition;
(ii) feeding the feedstock to a reactor;
(iii) heating the feedstock in the reactor, together with an acid-catalyst, to a first elevated temperature, to form an acid-treated mixture;
(iv) adding a neutralizing agent to the acid-treated mixture to form a neutralising-agent-treated mixture having a pH of at least 6;
(v) heating the neutralising-agent-treated-mixture to a second elevated temperature of at least 130ºC, preferably at least 160ºC, and most preferably about 170ºC, to form a further mixture;
(vi) optionally, filtering and/or dehydrating the further mixture obtained from step (v);

wherein the method comprises extracting volatiles from the reactor during steps (iii) and/or (iv) and/or (v), and
wherein the acid-catalyst is mixed with the feedstock:
   a. before heating of the feedstock, and/or
   b. during heating of the feedstock, and/or
   c. after heating of the feedstock; and
wherein:
   (A) in step (v) the further mixture is held at the second elevated temperature for less than 40 minutes, and optionally wherein
   (B) in step (v) the further mixture is maintained under air, and/or
   (C) in step (iv) the neutralising-agent-treated mixture has a pH of from 6 to about 7;
      wherein the reactor is operated:
      (a) under air, and/or
      (b) at partial vacuum of from 10 to 100 kPa.

With regard to step (i), the feedstock comprises waste biomass and a glycerol- or glyceride-containing composition. Such a composition provides the main chemical components for formation of the polyol. Glycerol is a known compound, and glyceride-containing compounds are also widely known. The most common glyceride-containing component is the triglyceride, found in oils and fats. The glycerol- or glyceride- containing composition acts as both a reactant and a solvent. In particular, a portion of the glycerol- or glyceride- containing compounds will undergo conversion and form a part of the final one or more biopolyols. However, a considerable amount of the glycerol- or glyceride-containing composition will act as the solvent for the waste biomass.

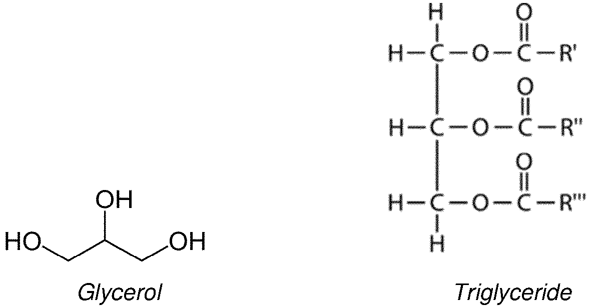

Waste biomass is a known type of material that is a plant or animal-based material, and which can be used as fuel (e.g. for electricity or heat production). By "waste", it is generally meant that the material may otherwise not be utilized, and would simply be discarded due to their low heating value, recalcitrance to typical conversion techniques, high moisture content and high fraction of inorganics. Waste biomass comprises macromolecules such as hemicellulose, cellulose, lignin proteins, and lipids that undergo decomposition. Example waste biomass includes wood-based material, crops, and waste from forests, yards or farms, as well as organic waste from water treatment or industrial plants.

The relevant amounts of the glycerol- or glyceride- containing composition and the waste biomass in the feedstock can vary significantly, and are dependent upon the available sources of each component at the time. In particular, the sources of biomass can be obtained based on the demand for biopolyols, and the material that is available within a logistically acceptable distance from the reaction site.

The preferred amount of the glycerol- or glyceride- containing composition in the feedstock is from 50 to 99 wt.%, preferably from 60 to 98 wt.%, more preferably from 75 to 95 wt.%, even more preferably from 85 to 92 wt.%, and most preferably about 90 wt.%, by combined weight of the waste biomass and the glycerol- or glyceride- containing composition. The preferred amount of the waste biomass is from 1 to 50 wt.%, preferably from 2 to 40 wt.%, more preferably from 5 to 25 wt.%, even more preferably from 8 to 15 wt.%, and most preferably about 10 wt.%, by combined dry weight of the waste biomass and the glycerol- or glyceride-containing composition.

Step (ii) includes feeding the feedstock to a reactor. Such reactors are known in the field of biopolyol manufacturing, and include continuously-stirred tank reactors and continuous plug flow reactors. Such reactors can be readily purchased from companies including Paul Mueller Company (USA), Zanon Pressure Equipment S.R.L. (Italy) and Fluitec (Switzerland). Preferably, the reactor has a stirring means that ensures that the contents of the reactor are continuously stirred/mixed.

Step (iii) includes heating the feedstock from step (ii) in the reactor, together with an acid catalyst, to a first elevated temperature, to form an acid-treated mixture. Different methods of heating the reactor can be employed. The skilled person is aware of various methods commonly used in the field. In addition, the most preferred method of heating the reactor contents can depend on the chosen type of reactor, and may include heating via thermal oil, steam, including medium pressure steam, an electric element, and/or microwaves. Preferably, medium pressure steam is used, as it works effectively in a full-scale setup. However, the use of microwaves to heat the reactor is also a preferred method, but the current cost of microwave heated reactors makes this method economically unfavourable. During this step, water vapour is preferably extracted/vented from the reactor to result in a mixture of dry reactants. The water vapour is considered a component of the "volatiles". The composition resulting at the end of step (iii) can be referred to as an "acid-treated mixture" or a "catalyst-containing mixture".

The process also includes that the acid catalyst is mixed with the feedstock: (a) before heating of the feedstock, and/or (b) during heating of the feedstock, and/or (c) after heating of the feedstock. The catalyst and feedstock may be added simultaneously or sequentially to the reactor. The catalyst may be mixed with the feedstock after the feedstock has already been added to the reactor, or the catalyst may be mixed with the feedstock before the feedstock is fed to the reactor (and therefore the catalyst-containing feedstock is subsequently added to the reactor), or the catalyst may already be present in the reactor, and then the feedstock is added to the reactor and the feedstock and catalyst are mixed within the reactor.

The catalyst is typically added in a predetermined amount relative to the dry weight of the initial glycerol- or glyceride- containing composition, and using known techniques to mix the catalyst evenly into the first mixture. The catalyst may be added manually or automatically. Preferably, the catalyst is added in a solution form, but can be added in solid form. Preferably, the mixture is first heated, and then the catalyst is added to the heated mixture. Then, after adding the catalyst, the mixture is preferably continuously heated to maintain the elevated temperature of the contents of the reactor, to allow the reaction to proceed.

Step (iv) includes adding a neutralizing agent to the acid-treated mixture to form a neutralising-agent-treated mixture having a pH of at least 6. This step is carried out in order to obtain a neutral pH (i.e. a pH of from 6 to 11, preferably from 7 to 8, and most preferably of about 7) after addition of the catalyst and the reaction has taken place. Neutralizing the mixture is important, as such a pH of from 6 to 11 is most preferred for polyurethane synthesis.

Step (v) includes heating the neutralising-agent-treated-mixture to a second elevated temperature of at least 130ºC, preferably at least 150°C, more preferably at least 160°C, and most preferably about 170°C, to form a further mixture. Such a step is done to allow the reaction to further proceed. The second elevated temperature is preferably less than 220°C, and more preferably less than 200°C, because the higher temperatures cause thermal decomposition of biomass constituents, which is undesirable.

Step (vi) is an optional step that includes further treating the mixture from step (v) by filtering and/or dehydrating the mixture. The mixture that results from the process of steps (i) to (vi) can be stored directly, ready for use in further reactions (e.g. reactions to make polyurethanes). Preferably, the mixture is filtered to remove unwanted sediments and solid particles that are not desired when further reacting the solution to produce polyurethane. The decision of whether or not to filter the mixture largely depends on the intended use of the mixture. For example, the one or more biopolyols in the form of suspensions might not be suitable for the desired use, and filtration may be a necessary processing step in the overall production scheme.

Different types of filtration can be used, such as decanting (i.e. gravimetrical separation/gravity filtration), vacuum-assisted filtration, centrifugal filtration, bag filtration, cartridge filtration, membrane filtration, chill/cold or hot filtration, multi-layer filtration, distillation, winterisation (also known as fractionate crystallisation) or none at all.

The solid filtration residues include centrifugation sludge and filtration cake, and can be collected manually or automatically upon cleaning of the centrifuges and filters in between production batches, or by any other appropriate method. The filtered mixture can then be stored or used immediately for a desired purpose. Storing of the one or more biopolyols can be done by putting the resulting biopolyol product mixture into empty pre-weighed barrels, containers, tanks, or trucks directly, depending on the desired scale. The relevant containers may then be sealed.

Dehydration can also be used as a post-treatment step, and is useful to remove any water formed upon neutralisation. The water formed can be evacuated, and the extent of dehydration depends on the intended use. For example, additional dehydration may be required if the one or more biopolyols are intended to be used in polyurethane synthesis.

Other post-treatment techniques can also be used, such as liquid-to-liquid extraction or vacuum distillation in order to further enhance the selectivity for key biopolyol fractures and corresponding parameters (such as viscosity, molecular weight etc.), depending on the requirements of the final product.

In addition, the method comprises extracting volatiles from the reactor during steps (iii) and/or (iv) and/or (v), and preferably during steps (iii) and (iv), and most preferably during each of steps (iii), (iv) and (v). In particular, during the heating step, and when adding the catalyst, and then the neutralising agent at an elevated temperature, the reaction results in volatiles boiling off and forming a gaseous phase within the reactor. This gaseous phase is preferably continuously vented during the steps where the mixture is hot or being heated. The volatiles comprise mainly of water and low amounts of organics, such as amines. Since the volatiles generally contain water, the step of extracting volatiles can also be considered as a drying or dehydrating step.

Surprisingly, the present inventors have found that the use of a feedstock comprising waste biomass and a glycerol- or glyceride- containing composition in a process according to the invention enhances the biopolyol product characteristics, and enables the production of high-quality biopolyols at mild reaction conditions (i.e. short retention time, partial vacuum or atmospheric pressure, in air, and at low reaction temperatures). The resultant biopolyols (either with or without filtration) were surprisingly found to be suitable for direct implementation in the synthesis of several polyurethane end-use products. In comparison, the processes known from the prior art use techniques requiring inert atmospheres, elevated pressures, higher reaction temperatures, much longer reaction times, excessive post-treatment of the products, and/or the use of impractical means of heating. The claimed process steps and starting feedstock together allow for an improved process, that is more simple, and has less environmental impact, due to lower energy usage (in the form of lower reaction temperature, duration and pressure), is able to use renewable reactant sources of a low quality, and a shorter reaction time.

Having the further mixture being held at the second elevated temperature for less than 40 minutes, preferably for 0 minutes (i.e. immediately allowing it to start cooling, or actively cooling) is advantageous because there is a reduced energy cost in the process.

In step (v) the further mixture is preferably maintained under air. This is advantageous because there is a reduced energy cost in the process associated with not requiring an inert or controlled gas environment. Preferably, steps (iv) and (v) are performed under air. This allows a reduced energy cost whilst unexpectedly allowing the reaction to proceed with a good conversion ratio.

In step (iv) the neutralising-agent-treated mixture preferably has a pH of from 6 to about 7, or more preferably about 7, most preferably 7. This has a material cost saving, since the mixture does not need to be over treated to observe the benefits of the invention.

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In one embodiment, the one or more biopolyols has a moisture content of less than 3 wt.%, or less than 2 wt.%, preferably less than 1 wt.%, and most preferably less than 0.5wt.%; and/or a hydroxyl number of at least 75 mgKOH/g, or at least 100 mgKOH/g, or at least 150 mgKOH/g, or at least 250 mgKOH/g, and/or an acid number of less than 30 mgKOH/g, or less than 20 mgKOH/g, or less than 15 mgKOH/g, preferably less than 10 mgKOH/g, and most preferably less than 5 mgKOH/g, and/or a moderate dynamic viscosity of up to 20 Pa.s, preferably up to 15 Pa.s, and most preferably up to 10 Pa.s at 25°C.

The claimed method is able to advantageously provide such a composition comprising one or more biopolyols having a low moisture content. The low moisture content enables the precise control of subsequent polyurethane synthesis. In addition, the process of the invention yields one or more biopolyols having a high hydroxyl number, which provides a high reactivity of the one or more biopolyols in subsequent polyurethane synthesis reactions.

In addition, the method of the present invention allows the acid number of the resulting one or more biopolyols to be maintained at a low value, meaning that the plasticizing effect of the acid groups is minimized. The dynamic viscosity of the resulting one or more biopolyols is also maintained low, meaning that the biopolyols are suitable for use in many polyurethane formation methods, including spray polyurethane application.

Preferably, the one or more biopolyols has a moisture content of less than 3 wt.% and a hydroxyl number of at least 75 mgKOH/g, and an acid number of less than 30 mgKOH/g, and a moderate dynamic viscosity of up to 20 Pa.s at 25°C. More preferably, the one or more biopolyols has a moisture content of less than 2 wt.% and a hydroxyl number of at least 100 mgKOH/g, and an acid number of less than 20 mgKOH/g, and a moderate dynamic viscosity of up to 15 Pa.s at 25°C. Even more preferably the one or more biopolyols has a moisture content of less than 1 wt.% and a hydroxyl number of at least 150 mgKOH/g, and an acid number of less than 15 mgKOH/g, and a moderate dynamic viscosity of up to 15 Pa.s at 25°C. Most preferably, the one or more biopolyols has a moisture content of less than 0.5 wt.% and a hydroxyl number of at least 250 mgKOH/g, and an acid number of less than 5 mgKOH/g, and a moderate dynamic viscosity of up to 10 Pa.s at 25°C.

The moisture content of the one or more biopolyols is measured according to DIN 51777; the hydroxyl number of the one or more biopolyols is measured according to DIN 53240; the acid number of the one or more biopolyols is measured according to DIN 53402; and the dynamic viscosity of the one or more biopolyols is measured according to DIN 53015.

Preferably, the reactor is a continuously-stirred tank reactor (CSTR) or a continuous plug flow reactor. The CSTR allows for the process to be carried out in batches, and the continuous plug flow reactor allows the process to be carried out as a continuous process. The CSTR advantageously keeps the mixture well homogenised throughout the process and can handle suspensions of high solid fractions. The CSTR is therefore the preferred type of reactor.

Preferably, the waste biomass comprises:
(a) sewage, preferably in the form of sludge, preferably with a dry matter content of at least 10 wt.%; and/or
(b) spent mushroom substrate, preferably with a dry matter content of at least 50 wt.%; and/or
(c) municipal organic waste, preferably comprising food waste and/or paper and/or cardboard and/or gardening waste, and wherein the municipal organic waste preferably has a dry matter content of at least 25 wt.%.

The sewage may be in the form of a sludge, or a slurry, or as dry matter. Most preferably, the sewage is dry, but in reality, it is challenging to obtain sewage in a dry form, and so sewage sludge is mostly used. The sewage sludge is preferably digested sewage sludge, which is the product of either aerobic or anaerobic digestion of the sewage, and is a well-stabilized material capable of being dewatered.

The spent mushroom substrate is also known as spent mushroom compost, and comprises, consists essentially of, or consists of, the residual biomass waste generated by the mushroom production industry. Spent mushroom compost typically contains significant amounts of cellulose and lignin, with some xylose (a key hemicellulose) and a high fraction of inorganics (often >30% on a dry matter basis). In general, it is a high moisture content biomass (20-50% DM).

Municipal organic waste includes food waste and/or paper and/or cardboard and/or gardening waste and/or farming waste and/or macroalgal waste. Generally, municipal waste is any form of cellulose-based waste that would otherwise be discarded. This can also include garden or farming waste, such as crops, or parts of crops. It can also include by-products from extraction at biorefineries, macroalgal waste, fishing waste, and food production waste. One example includes hemp stalk hurds, which are the hurd fibers in the stalks of hemp plants, and have a high cellulose content. Other examples are sugar beet pulp, which is a fibrous material left over after the sugar is extracted from sugar beets, and decomposing seaweed collected at ports, beaches or on marine vessels.

The waste biomass may contain any combination of one or more of (a) to (c), which are all abundantly available, and would otherwise be disposed of. Most preferably, the waste biomass comprises (a) sewage, and preferably digested sewage sludge. The waste biomass can contain any relative amounts of (a) to (c), as these components can be easily mixed together to form an appropriate waste biomass for the feedstock.

The minimum dry matter contents recited for each of components (a) to (c) are preferred because the more moisture present in the original biomass, and therefore in the feedstock, the more energy is required, and consumed during the reaction, in order to reduce the moisture content in the final composition comprising one or more biopolyols. In addition, a lower water content in the waste biomass, and therefore the feedstock, means that less wastewater management processes are required, making the logistics of the process easier and more sustainable.

Preferably, the sewage has a dry matter content of at least 20wt.%, more preferably at least 30 wt.%, even more preferably at least 40wt.%, and most preferably at least 50 wt.%.

Preferably, the spent mushroom substrate has a dry matter content of at least 60 wt.%, more preferably at least 70 wt.%, and most preferably at least 80 wt.%.

Preferably, the municipal organic waste has a dry matter content of at least 25 wt.%, more preferably at least 40 wt.%, even more preferably at least 50 wt.%, and most preferably at least 60 wt.%.

Although a higher dry matter content (i.e. a lower water content) is generally preferred for each source of the waste biomass, the maximum dry matter content of the sources is limited due to the format in which the waste biomass exists/can be obtained. For example, sewage is most commonly obtainable in a sludge format, as opposed to a solid format, and both food (including mushrooms) and garden/farm waste have a substantial pre-existing water content, due to the nature of the organic substances.

Preferably, the glycerol- or glyceride-containing composition comprises glycerol, and/or vegetable oil, preferably used/waste cooking oil, preferably wherein the glycerol- or glyceride-containing composition comprises, or consists essentially of, or consists of, glycerol, most preferably crude glycerol, the crude glycerol preferably having less than 3 wt.% ash content. By oil, it is meant any kind of oil including wastewater treatment grease, and oils from soya, castor, palm, palm kernel, canola (rapeseed), peanut, cottonseed, coconut, olive, algae and sunflower. More preferably, the oil is a waste/used cooking oil, and the waste cooking oil acts as a source of glyceride, and comprises a high and majority amount of triglycerides, composed of different fatty acids. Most preferably, the glycerol- or glyceride-containing composition comprises, or consists of, crude glycerol. Crude glycerol is the most preferred glycerol-containing composition, as it has proven to be highly effective at acting as both a reactant (undergoing polycondensation) and as a solvent (breaking down the bonds in the waste biomass in the initial stages of the conversion process, partially liquefying it).

Waste cooking oil is still effective at acting as a reactant in the mixture, but is not as effective as crude glycerol as also acting as a solvent. Although waste cooking oil can often be sourced at a relatively low price (making it appear as an economically viable option for the glyceride-containing composition), in reality, the conversion of the waste cooking oil is more cost-intensive than the conversion of crude glycerol and waste biomass, and so the costs and convenience balance out, such that both crude glycerol and waste cooking oil are viable options.

Crude glycerol is a low-value by-product primarily obtained from the biodiesel production process. Its composition is different from that of pure glycerol, as it contains various impurities, such as water, methanol, soap, salts, fatty acids, fatty acid methyl esters, glycerides and methanol. Crude glycerol is preferred to due being of low cost, and abundantly available. Crude glycerol generally comprises at least 50 wt.% glycerol, preferably at least 75 wt.% glycerol. Crude glycerol generally has up to 10 wt.% non-glycerol organic matter, preferably less than 5 wt.% non-glycerol organic matter, and generally has less than 5 wt.% ash, most preferably less than 2.5 wt.% ash. Preferably, the crude glycerol contains a high concentration of the polyhydric alcohol. Crude glycerol performs better in the process of the invention than pure glycerol, because while most impurities exhibit antagonistic effects on the conversion process, the residual fatty acid methyl esters in crude glycerol are known to benefit the simultaneously occurring liquefaction.

Preferably, the feedstock is pre-mixed before being sent into the reactor in step (ii). This ensures that the different components of the feedstock are adequately mixed before being heated, which allows for a more uniform heating of the feedstock. Preferably, step (iii) includes mixing the feedstock whilst heating, which also ensures more uniform heating of the feedstock (and the catalyst when this is added before or during heating).

Preferably, step (iii) includes heating to a first elevated temperature of from 110 to 170 °C, preferably from 110 to 155 °C, more preferably from 120 to 140 °C, most preferably about 130°C, and wherein once the catalyst-containing mixture has reached the first elevated temperature, the catalyst-containing mixture is held at the first elevated temperature for a period of from 20 mins to 3 hours, more preferably from 30 mins to 2 hours, and most preferably about 60 mins. Such a reaction time of the catalyst-containing mixture is most preferred to allow sufficient time for a good conversion ratio, while keeping the balance between energy input and conversion ratio.

The most preferred process includes holding the heated catalyst-containing mixture of step (iii) for 60 mins, in order to allow the reaction to progress, and then to add the neutralizing agent, increase the temperature further, and then cease the reaction (i.e. have a duration of 0 mins after the mixture containing the neutralizing agent has reached the second elevated temperature). The reaction is generally stopped after a time taken to reach the desired moisture level. This can be immediately, or after up to 2 hours, depending on the specific process conditions.

Preferably, as soon as the second elevated temperature is reached, the mixture is allowed to cool down naturally or via a heat exchanging step. The mixture may be subsequently passed through centrifuges and/or filters while the mixture is still warm. This is preferred as it takes advantage of the differences in densities. After the mixture has been allowed to fully cool, a further filtration process is preferably conducted, in order to remove any precipitates that form upon cooling.

The reactor can be operated under an inert atmosphere or under air, and is preferably operated under air, and/or at partial vacuum. By partial vacuum, it is meant a pressure of from 10 to 100 kPa, preferably 30 to 70 kPa, more preferably 40 to 60 kPa, and most preferably 50 kPa. Surprisingly, the inventors discovered that the process of the invention can operate under air, rather than under nitrogen, and still obtain high quality biopolyols at a decent conversion ratio. In addition, the use of a partial vacuum was surprisingly found to allow the reaction to proceed with a good conversion ratio, without requiring the energy and cost demands to operate under full vacuum.

Preferably, as soon as the second elevated temperature is reached, the mixture is held at the second elevated temperature for a period of from 0 to 15 mins preferably from 0 to 10 mins, and most preferably 0 mins, and the reactor is operated under air, and step (iii) includes adding the catalyst to the mixture in an amount of about 2 wt.%, by dry weight of the initial glycerol- or glyceride- containing composition, and step (iv) includes adding the neutralizing agent in an amount of from 1 to 2.5 wt.%, by weight of the catalyst-containing mixture present at the end of step (iii) to neutralize the mixture. Such a combination of process conditions is most preferable for producing a good quality bio-polyol product from a low quality feedstock, while maintaining a good conversion ratio. The step of adding the neutralizing agent in an amount to neutralize the mixture is typically done by adding sequential small portions of neutralizing agent to the mixture until the mixture is neutralized (i.e. pH of about 7). No further neutralizing agent is added after neutralization is reached, so the mixture will reach a final amount of no more than 0.25 wt.% neutralizing agent, preferably no more than 0.1 wt.% neutralizing agent and most preferably 0 wt.% neutralizing agent in the mixture.

Preferably, the acid catalyst is a strong acid with a pKa greater than 1.74, and more preferably wherein the acid catalyst comprises sulphuric acid and/or phosphoric acid and/or hydrochloric acid and/or nitric acid. The most preferred catalyst is sulphuric acid. The acid is preferably provided in solution form, but can be provided in solid form. Acid-catalysed conversion of chemical compounds into biopolyols is known to be more efficient (than not adding a catalyst) because the reaction can occur at lower temperatures and reach high conversion ratios much faster. There is a strong correlation between the strength of the acid and its catalytic performance. The acid accelerates how fast the chemical bonds are broken down, by reducing the activation energy to break such bonds. This results in the macromolecules making up the waste biomass (e.g. hemicellulose, cellulose, lignin, and proteins) getting reduced to lower molecular weight constituents (e.g. oligomers, polypeptides) more quickly, and requiring less energy input (due to the reduction in activation energy), than if the acid catalyst was not present.

Preferably, the acid catalyst is added to the mixture in an amount of from 0.5 to 5 wt.%, preferably from 1 to 3 wt.%, and more preferably about 2 wt.%, by dry weight of the initial glycerol- or glyceride- containing composition. This is the preferred amount of the catalyst to ensure that there is sufficient catalyst to adequately catalyse the product of biopolyols via solvothermal liquefaction. The amount of catalyst is measured relative to the dry weight of the initial glycerol- or glyceride- containing composition. In particular, during the subsequent steps within the reactor, it is practically too challenging to calculate the amount of catalyst relative to the amount of the heated mixture, and so an appropriate amount of catalyst can be easily and reliably calculated based on the amount of initial feedstock. In addition, since the water content of the initial glycerol- or glyceride- containing composition can vary, the appropriate amount of catalyst for the reactants can be more accurately measured in relation to the dry mass. "By dry weight" is considered to be relative to the weight of the glycerol- or glyceride- containing composition having less than 5 wt.% water. The amount of catalyst supplied to the system in step (iii) is known to plateau in terms of its catalytic effect. Therefore, amounts above 5 wt.% do not tend to provide a further gain in terms of increasing the reaction rate (i.e. a point of saturation is passed), and so there is no benefit to adding more than 5 wt.% of catalyst.

Preferably, step (iv) includes adding the neutralizing agent in an amount of from 0.2 to 5 wt.%, preferably 0.3 to 4 wt.%, and more preferably from 0.5 to 3 wt.%, and even more preferably from 1 to 2 wt.%, by weight of the catalyst-containing mixture present at the end of step (iii), which is also called the 'intermediary mixture'. Such an amount is the optimum required to provide the desired neutralizing effect, and provide a mixture with a desired pH. The step of adding the neutralizing agent is generally done by adding small portions to the reactor sample and measuring the pH, until the desired neutral pH is reached. The neutralizing agent is preferably a base, and is preferably selected from sodium hydroxide, potassium hydroxide, calcium hydroxide, sodium bicarbonate, or mixtures of two or more thereof; and is most preferably sodium hydroxide. Neutralization advantageously enables the removal of certain salts that would otherwise precipitate once the product is cooled. The salts may include sodium sulphate, sodium chloride, sodium stearate, and sodium oleate. In addition, the one or more biopolyols should have a pH of 7 to 11, in order to make them appropriate for subsequent use in reactions, including polyaddition reactions to make polyurethanes.

The neutralizing agent may be added in an amount to both neutralize the mixture, and also catalyse the reaction further (i.e. by adding more than the amount required to neutralize the mixture). When additional neutralizing agent is added to catalyse the reaction, the final amount in the mixture is from 0.3 to 1 wt.%, or preferably 0.5 wt.%. However, the neutralizing agent may be added in only an amount required to neutralize the mixture, so that the final mixture contains only up to about 0.25 wt%, preferably up to about 0.1 wt.%, and more preferably about 0 wt.% neutralizing agent. When additional neutralizing agent is added to catalyse the process, about 2 to 4 wt.%, preferably 2.1 to 3.1 wt.% neutralizing agent is added. When only enough neutralizing agent is added to obtain a neutral pH, without adding further for catalysis, an amount of about 1 to 2wt.%, preferably 1.4 to 1.8 wt.%, and most preferably about 1.6wt.% is used.

Preferably, the liquid to solid ratio of the feedstock is from 15:1 to 3:1. The liquid to solid ratio is highly dependent on what kind of feedstock is acquired, which is also tied into the changing markets, and the waste biomass that is available at the time that it is required. More preferably, the liquid to solid ratio is at least 5:1. This liquid to solid ratio can most readily be achieved through use of high-quality crude glycerol and relatively dry sewage. In contrast, when a mix of crude glycerol, waste cooking oil, and sewage sludge is used, then the liquid to solid ratio is generally around 10:1. Thus, the liquid to solid ratio is preferably from 12:1 to 4:1.

Preferably, the combination of steps (iii) to (v) have a duration of from 30 minutes to 6 hours, preferably from 30 minutes to 5 hours, or more preferably from 60 minutes to 4 hours. This amount of time allows the reaction to reach a desirable conversion ratio of the waste biomass, and allows sufficient time for the vapour and volatiles to be evacuated, leaving the one or more biopolyols with a desirably low moisture content.

Although longer reaction times lead to increased conversion ratios, particularly long reaction times have diminishing returns due to the equilibrium shifting and favouring recondensation reactions. Thus, after an initial increase in the conversion ratios, hydroxyl numbers, and a decreased viscosity, an inverse trajectory is observed after a certain length of time, which can vary slightly, depending on the specific waste biomass used. In general, a total reaction time over steps (iii) to (v) of less than 6 hours is used.

Preferably:
(a) the catalyst is added to the feedstock before heating, and the mixture of catalyst and feedstock is heated to a temperature of from 110 to 170°C, and then held at this temperature for a period of from 15 minutes to 5 hours; or
(b) the catalyst is added to the feedstock during heating, and the mixture of catalyst and feedstock is heated to a temperature of from 110 to 170°C, and then held at this temperature for a period of from 15 minutes to 5 hours; or
(c) the catalyst is added to the feedstock after heating, and the mixture of catalyst and feedstock is held at a temperature of from 110 to 170°C for a period of from 15 minutes to 5 hours. More preferably, in each of situations (a) to (c), the mixture is held at a temperature of from 120 to 160 °C, or 130 to 150°C, for a period of from 30 minutes to 4 hours, more preferably from 30 minutes to 3 hours, and more preferably the mixture is held at 130 to 150°C, for from 45 minutes to 2 hours.

These scenarios provide the preferred time period for the catalyst-containing feedstock to be held at an elevated temperature, allowing the reaction to proceed sufficiently to give a desired yield of one or more biopolyols.

Preferably, the pressure is maintained at from 10 kPa to 100 kPa, more preferably from 30 to 80 kPa, and even more preferably at about 50 kPa. This pressure provides a partial vacuum that encourages unwanted volatiles to boil off the mixture and be removed.

In a further aspect not claimed, there is provided a composition comprising biopolyols produced by the method as described herein. The biopolyols produced using the method described can be used in standard polyurethane production processes, and are produced from a sustainable source, as opposed to finite fossil raw materials.

The invention will now be described in relation to the following non-limiting figures, in which:
- Figure 1 shows an overview of a process according to the invention.
- Figure 2 shows a detailed scheme of a process according to the invention.
- Figures 3A to 3D show enlarged versions of different sections of the process scheme of Figure 2.
- Figures 4A to 4D show the Fourier transform - Infrared spectra of biopolyols produced using different feedstocks. The wave number in cm⁻¹ is depicted on the x-axis, and the relative absorbance, in %, is depicted on the y-axis.
- Figure 5 shows the conversion ratio, in % on the y-axis, for different test runs of the same D3 feedstock under different process conditions, which are displayed on the x-axis.
- Figure 6 shows the hydroxyl number, in mgKOH/g on the left y-axis, and acid number, in mgKOH/g on the right y-axis, for each different test run under different process conditions, which are displayed on the x-axis.
- Figure 7 shows the hydroxyl number, in mgKOH/g on the y-axis, for biopolyol products produced from various feedstocks under two different process conditions, both straight after being processed, and after being filtered.

### DETAILED DESCRIPTION

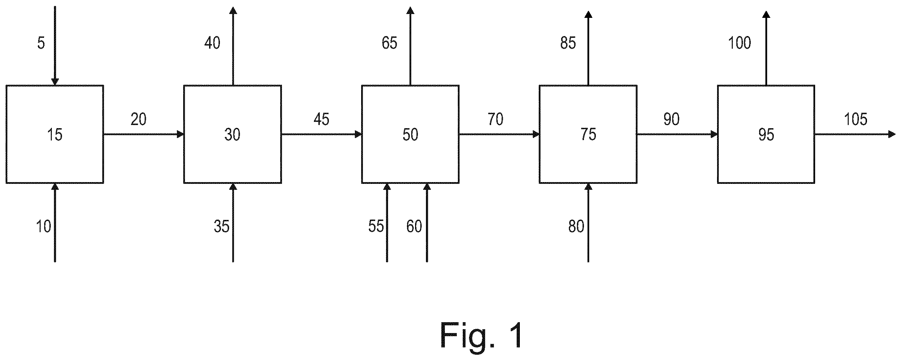

With regard to Figure 1, the exemplary process includes feeding crude glycerol 5 and waste biomass 10 into a mixer/homogenizer 15 to form a feedstock 20. The feedstock 20 is then fed into a continuously-stirred tank reactor 30, where heat 35 is applied to heat the contents of the reactor to a temperature of from 110 to 170°C. During this heating step, vapour and low boiling compounds 40 are extracted.

The resulting dry reactants 45 are further processed by adding an acid catalyst 55 to form a mixture 50 of heated feedstock and catalyst. Further heat 60 is applied to the mixture 50 to catalyse the solvothermal liquefaction reaction. Volatiles 65 are extracted from the reactor during the solvothermal liquefaction reaction. The resulting intermediary biopolyols 70 are then neutralized by adding a base agent 80 to form a neutralized mixture 75. Volatiles 85 are further vented from the reactor during the neutralisation step. The resulting biopolyol mixture 90 is then passed to a unit 95 for further processing, which results in the removal of by-products 100 by filtration, to yield the final biopolyol product 105.

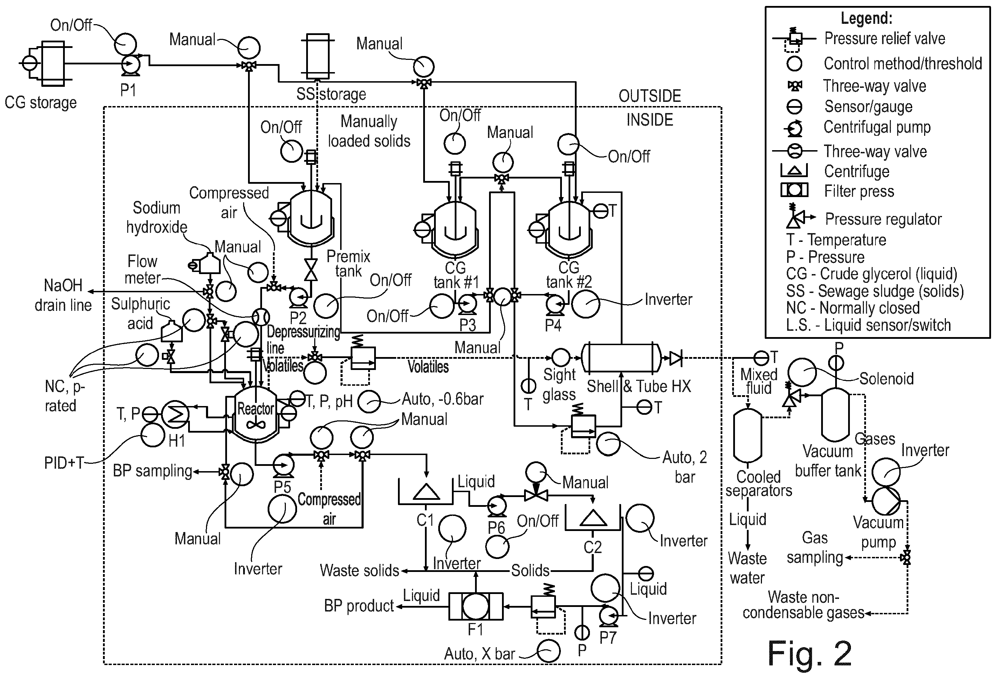

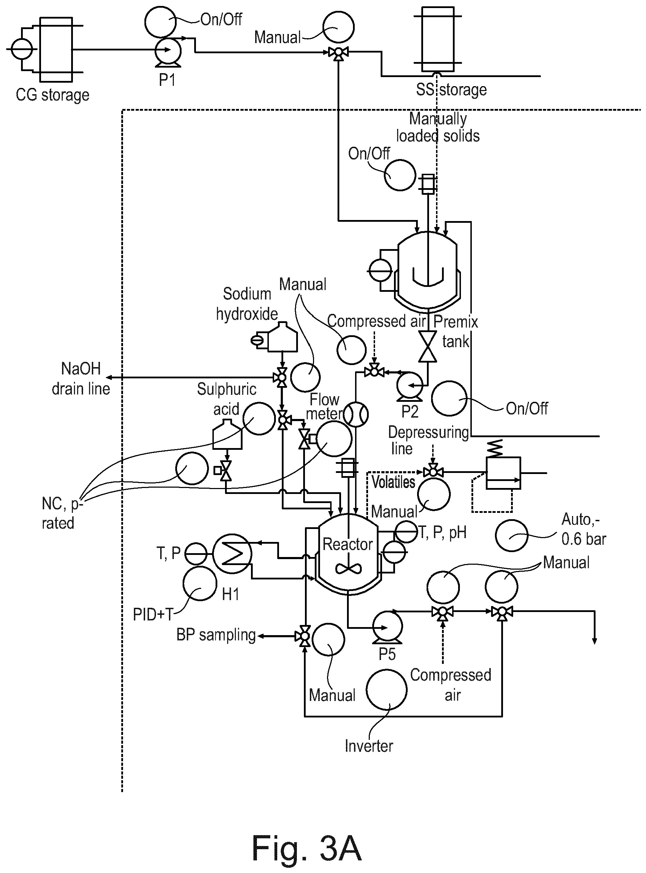

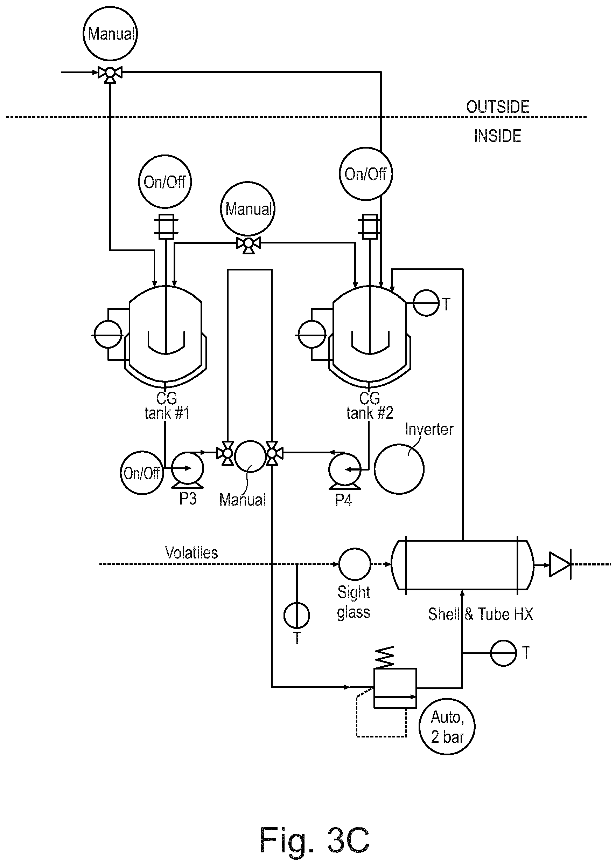

With regard to Figure 2, and in particular to Figure 3A, a crude glycerol composition is pumped by barrel pump P1 from its storage container to a 3-way valve, where the composition is split into two streams. A first stream is sent to a further 3-way valve (shown in Figure 3C), and a second stream is sent to a premix tank. The premix tank is a thermally insulated storage tank.

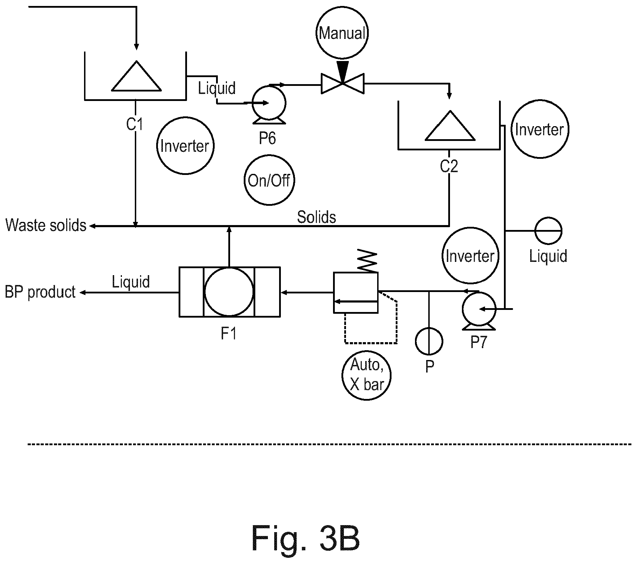

In the premix tank, sewage sludge is added from where it is stored to the pre-mix tank to mix with the crude glycerol, and form the feedstock. The sewage sludge is transported to the premix tank via a means that is dependent on the phase of the feedstock. When the sewage is solid, a manual or conveyor is used, and when the sewage is in slurry form, a pump is used. Further crude glycerol can be added from tank #1 or #2, shown in Figure 3B.

The homogenised mixture from the premix tank is pumped by centrifugal pump P2 to a further 3-way valve. Compressed are is used to purge the line and avoid blockages in the pipe. The mixture is then passed to a reactor, while the flow rate is measured by a flow meter to ensure the correct rate of addition into the reactor. The reactor has an element that continuously stirs the contents therein. The reactor is also jacketed.

In the reactor, heat is applied by heat exchanger H1, to heat the contents of the reactor to a temperature of from 110 to 170 °C. At the same time, a partial vacuum of 50 kPa is applied to enhance evaporation of the volatiles. Since the evaporation of the volatiles includes loss of water, the step can also be considered as a drying step (i.e. dehydration).

Sulphuric acid solution, in an amount of 2 wt.%, by dry weight of the initial crude glycerol, is then added after dehydration, whilst the elevated temperature is maintained, to catalyse the solvothermal liquefaction reaction in the reactor. The acid catalyst is added from where it is stored in a container tank that is mounted directly above the reactor. After a period of about 60 minutes after addition of the catalyst (the period including continued heating to maintain the temperature at from 110 to 170°C), sodium hydroxide base is added to neutralize the reactants. The contents of the reactor are then removed for further processing.

The sodium hydroxide is first added to a container tank that is mounted directly above the reactor. Then, the sodium hydroxide is supplied gravimetrically to the reactor. During the steps performed in the reactor, the temperature, pressure and pH are monitored by sensors. The sodium hydroxide is supplied in a controlled manner using a valve until the contents of the reactor reach a neutral pH (i.e. pH of approximately 7). The amount of sodium hydroxide required is generally approximately 1 to 3 wt.% of the initial dry mass of crude glycerol.

The mixture is then heated to a further elevated temperature that is at least 130°C and dried under vacuum for another approximately 15 minutes, to allow the volatiles to be removed.

During the acid-catalysed reaction, before the neutralization agent is added, volatiles are generated and are extracted, cooled and condensed in the shell and tube heat exchanger and the cooled separators. In addition, during this process the pressure in the reactor, heat exchanger HX1 and separators is maintained at a pressure of about 50 kPa by a vacuum pump and vacuum buffer tank.

With further reference to Figure 3B, the resulting warm mixture is then pumped by P5 for centrifugation in centrifuge C1. The centrifuge C1 separates the solid and liquid, and the waste solids are removed. The resulting liquid is then pumped by P6 to a second centrifuge C2, where a further centrifugal force is exerted on the liquid to further separate the solid particles. The solids from C2 are combined with the waste solids from C1, and are stored prior to further utilization. The waste solids include centrifugation sludge, and filtration cake. The solid filtration residues are collected manually or automatically upon cleaning of the centrifuges and filters in between product batches. The liquid from C2 is pumped by P7 through a pressure relief valve, and then through a filter press, which is a fine mesh filter, to give the final biopolyol product.

The biopolyol product comprises one or more biopolyols, and is stored in empty and pre-weighed barrels, and sealed.

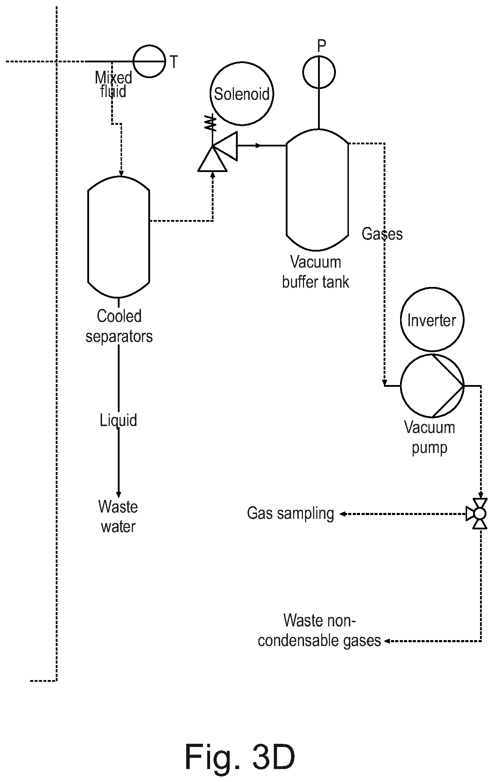

Turning back to Figure 3A, during the heating step in the reactor, before and after acid addition, volatiles are vented and sent through a depressurising line and a pressure relief valve. With reference to Figure 3C, the resulting volatiles are sent past a temperature sensor, through sight glass, and into a shell and tube heat exchanger. The volatiles are cooled by heat exchange with the crude glycerol from tanks #1 and #2, and the resulting cooled mixed fluids are sent, via a temperature sensor, to liquid cooled separators (see Figure 3D). The volatiles are collected in the separators and stored prior to subsequent wastewater treatment.

With reference to Figure 3D, liquid exits the cooled separators at the bottom, to give waste water, and gases pass through a pressure regulator into a vacuum buffer tank. The pressure in the vacuum buffer tank is monitored, and the resulting gases are then passed via a vacuum pump for gas sampling or as waste non-condensable gases.

Turning back to Figure 3C, a portion of the crude glycerol from the crude glycerol storage container is passed via a 3-way valve, to either a tank #1 or tank #2. These tanks are used as storage tanks for the crude glycerol. The crude glycerol is then pumped by P3, using a 3-way valve, either back around into tank #1 or #2, or to the premix tank. Some of the crude glycerol is pumped into tank #2. Then, from tank #2, the crude glycerol is pumped by P4 via a 3-way valve, either back into tank #1 or #2, or to the shell and tube heat exchanger, where the crude glycerol is heated by the hot volatiles from the reactor to a temperature of approximately 50°C.

The invention will now be described in relation to the following non-limiting examples.

### EXAMPLES

### Reference Example 1

Various feedstock samples were processed to yield a product comprising one or more biopolyols. The resulting product of one or more biopolyols produced from each feedstock sample was then analysed using Fourier-transform infrared spectroscopy (FT-IR).

The feedstock samples are labelled with a letter, D, S or H, which denotes the source of biomass, and a number, 1, 2, 3, 4 or 5, which denotes a particular combination of waste biomass particle size and dry matter content. Each feedstock uses crude glycerol as the glycerol- or glyceride- containing composition.

B0 is a blank, comparative feedstock, comprising only crude glycerol, without any waste biomass.

The letters D, H and S have the following meaning:
- D - the waste biomass in the sample is digested sewage sludge. The total feedstock is made up of crude glycerol and the digested sewage sludge, with an initial liquid to solid ratio of 10:1.
- H - the waste biomass is hemp stalk hurds. The feedstock comprises crude glycerol and the hurds, and has an initial liquid to solid ratio of 10:1.
- S -the waste biomass is sugar beet pulp. The feedstock comprises crude glycerol and the sugar beet pulp, having an initial liquid to solid ratio of 10:1.

The numbers 1 to 5 signify the particle size and dry matter content of the waste biomass as such:
- 1 - dry matter content of 100%, and a particle size of <0.2 mm.
- 2 - dry matter content of 100%, and a particle size of 0.2 to 0.5 mm.
- 3 - dry matter content of 100%, and a particle size of 0.5 to 1 mm.
- 4 - dry matter content of 50%, and a particle size of 0.5 to 1 mm.
- 5 - dry matter content of 20%, and a particle size of 0.5 to 1mm.

The three powder particle size fractions (i.e. less than 0.1 mm, 0.2 to 0.5 mm, and 0.5 to 1 mm) were isolated using a vibrating test shaker EML Digital Plus with standard sieves from Haver and Boecker (classification as per DIN 11540).

The process was carried out according to the present invention, under the following reaction conditions:
- Catalyst used: 98% aqueous sulfuric acid, in an amount of 3 wt.%, by weight of the initial crude glycerol;
- Reaction time (i.e. time reaction held at after heating to between 110 and 130°C and adding the catalyst): 60 minutes;
- Reaction temperature: For 20% dry matter, the reactor contents were heated to 110 °C, for 50% dry matter, the reactor contents were heated to 120 °C, and for 100% dry matter, the reactor contents were heated to 130°C.
- Reaction pressure: atmospheric.
- Neutralization agent: sodium hydroxide pellets; added after both addition of the catalyst and being held at an elevated temperature for a set duration. The amount is approximately 2.7 wt.%, by weight of the intermediary composition, in order to result in a final amount of 0.5 wt.% NaOH by weight of the intermediary composition.
- After neutralizing agent added, reactor contents heated to 170°C and then held for 45 minutes.
- Atmosphere during acid-catalysed reaction: air.
- Atmosphere during neutralization and dehydration: inert gas (N₂) (inert atmosphere added immediately before addition of neutralisation agent)
- Post-treatment: solvent (98% ethanol) assisted vacuum filtration with subsequent solvent evaporation via rotary vacuum distillation.

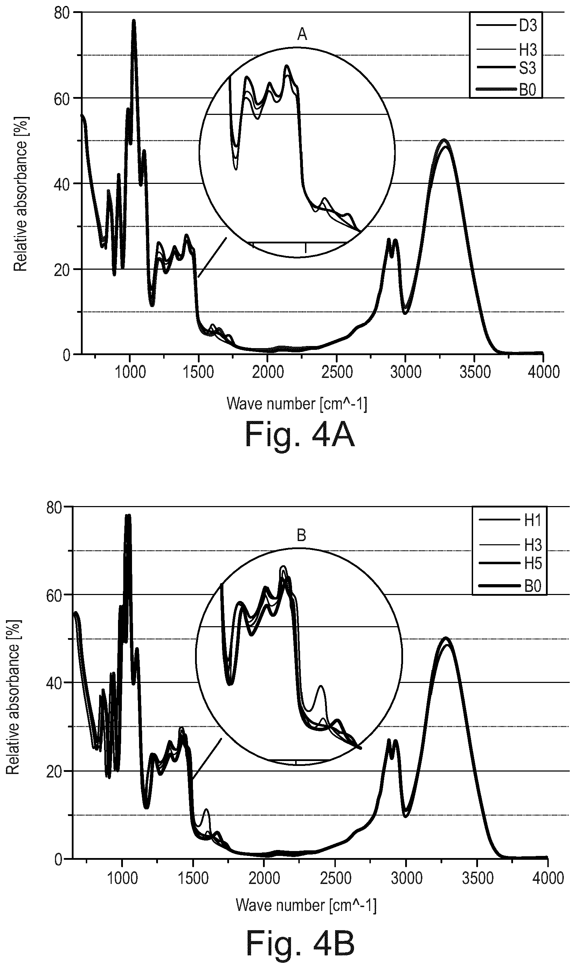

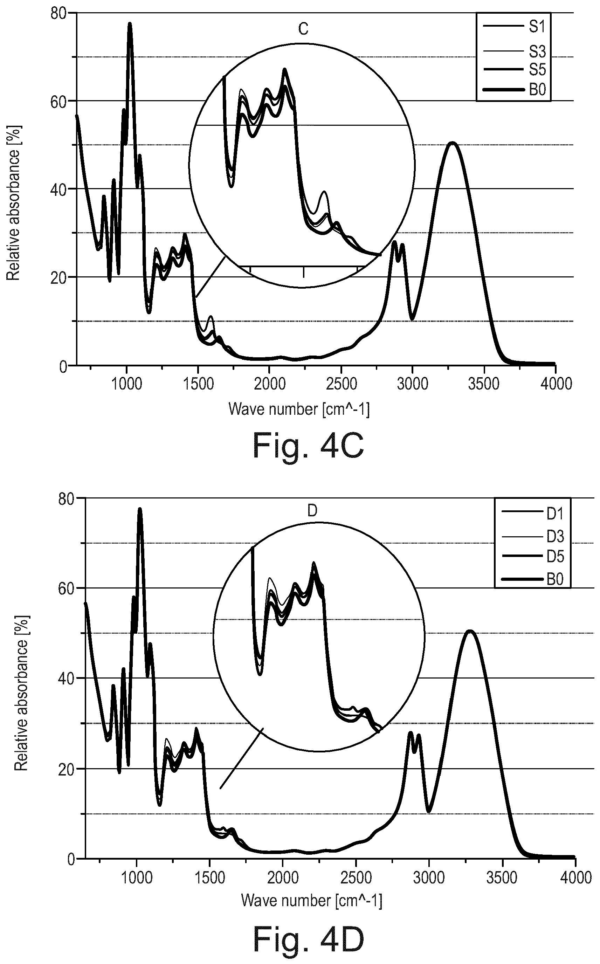

Figure 4A shows the FT-IR spectra of the biopolyol product sample resulting from processing the following feedstock samples: B0, D3, H3, and S3. Figure 4B shows the FT-IR spectra of the biopolyol product sample resulting from processing the following feedstock samples: B0, H1, H3, and H5. Figure 4C shows the FT-IR spectra of the biopolyol product sample resulting from processing the following feedstock samples: B0, S1, S3, and S5. Figure 4D shows the FT-IR spectra of the biopolyol product sample resulting from processing the following feedstock samples: B0, D1, D3, and D5. The FT-IR spectra were generated using a Perkin Elmer Frontier spectrometer.

### Reference Example 2

The feedstock samples B0, D1, D2, D3, D4, and D5, as described above for example 1, were subjected to the process described above for example 1. The resulting biopolyol product mixture was then analysed by gas chromatography - mass spectrometry (GC-MS), to identify the composition of one or more biopolyols resulting from the process, as well as any other by-products, including esters, ketones and carboxylic acids. The compounds present in the methanol soluble phase of the biopolyols were analysed on a GC-2010 Plus and QP2010 Ultra GCMS setup from Shimadzu. The analysis was done on a Rxi^{®}-5ms column (29 m x 0.25 mm 0.25 µm, made of crossbond^{®} 5% diphenyl / 95% dimethyl polysiloxane). Detector grade helium was the carrier gas with a constant column flow of 1.8 mL/min. The oven was programmed to an isotherm of 2 min at 50 ºC with a subsequent 10 ºC/min ramp to the final temperature of 250 ºC. The injection volume was 0.1 µL; solvent cut time was 3 min. The analysis was carried out in split mode at a 35 split ratio. The ion source and interface temperatures were held constant at 200 ºC and 260 ºC. The peak identification was based on computer matching of the mass spectra with the NIST 08 library. Prior to the analysis, the samples were diluted with methanol and filtered. Only successfully identified peaks were taken into account (i.e., distinct peaks and NIST match probabilities of at least 60%).

The symbol + indicates that the compound was identified in the product sample, and - indicates that the compound was not identified in the product sample. The results of the GC-MS analysis of the resulting product samples are shown below in Table 1.

**Table 1**

| **Retention time [min]** | **Compound** | **Database similarity [%]** | **B 0** | **D 1** | **D 2** | **D 3** | **D 4** | **D 5** |
|---|---|---|---|---|---|---|---|---|
| 4.35 | Glycerol 1-monomethyl ether | 98 | - | + | + | + | + | + |
| 5.14 | 1,3,4-Butanetriol | 88 | | - | - | - | + | + |
| 10.89 | Glycerol | 96 | + | + | + | + | + | + |
| 11.47 | Diglycerine | 75 | + | + | + | + | + | + |
| 12.01 | 2-Deoxyhexose | 85 | | - | + | + | + | + |
| 12.35 | Erythritol | 83 | - | - | - | - | + | - |
| 12.41 | Heptose | 78 | - | - | + | + | + | + |
| 12.47 | 1,2,3-Butanetriol | 82 | - | + | + | + | + | + |
| 12.61 | Diglycerine | 75 | + | + | - | - | - | - |
| 12.91 | 3,4-Altrosan | 84 | - | + | + | + | + | + |
| 13.06 | Propyl hexanoate * | 80 | + | + | + | + | + | + |
| 13.42 | Levoglucosan | 92 | - | + | + | + | + | + |
| 14.09 | Diglycerine | 80 | + | + | + | + | + | + |
| 14.65 | 1,4-Dioxane-2,5-dimethanol | 84 | + | + | + | + | + | + |
| 15.05 | Deoxyribose | 77 | - | + | + | + | - | - |
| 16.15 | 2-Deoxyhexose | 72 | - | + | + | + | + | + |
| 16.37 | 3-(Methylthio)-1,2-propanediol | 73 | - | + | + | + | + | + |
| 17.47 | Hexyl 3-phenylpropanoate * | 77 | - | + | + | + | + | + |
| 18.03 | Methyl hexadecanoate * | 84 | - | + | + | + | + | + |
| 18.37 | Hexadecanoic acid * | 90 | - | + | + | + | + | + |
| 18.74 | Polygalitol | 75 | - | + | + | + | + | + |
| 19.45 | Z,Z-8,10-Hexadecadien-1-ol * | 80 | - | + | + | + | + | + |
| 19.66 | Tridecanedial * | 83 | - | + | + | + | + | + |
| 19.72 | Methyl trans-9-octadecenoate * | 92 | - | + | + | + | + | + |
| 19.86 | 2-Hexadecanoyl glycerol | 90 | - | + | + | + | + | + |
| 20.06 | Oleic Acid * | 89 | + | + | + | + | + | + |
| 20.63 | 4-Nitro-5-hydroxy-1,2-dimethylindole * | 62 | - | + | + | + | + | + |
| 21.23 | Polygalitol | 61 | - | + | + | + | + | + |
| 21.5 | 2-Hexadecanoyl glycerol | 80 | - | + | + | + | + | + |

| | | | | | | | | |
|---|---|---|---|---|---|---|---|---|
| * Non-biopolyol by-products | | | | | | | | |

The results show that crude glycerol alone does react to form one or more biopolyols. However, the inclusion of a waste biomass source with the crude glycerol results in the production of more biopolyol products. The resulting product mixture also contains various non-biopolyol compounds. Most of these are impurities present in the crude glycerol starting material. The saturated long-chain fatty acids (e.g. hexadecenoic acid) and fatty acid methyl esters (e.g. methyl hexadecanoate) are common impurities in the crude glycerol. However, some of the non-biopolyol compounds are by-products that result from the reaction of the crude glycerol (and waste biomass, when present) in the solvothermal liquefaction reaction. These impurities can be compounds having only one OH group, carboxylic acids, esters, ketones, etc. Further filtration steps can be performed to remove the by-products and impurities. Such by-products and impurities make the resultant biopolyol mixture less reactive, and so further post-treatment may be necessary depending on the final use of the biopolyol mixture.

### Example 3

The feedstock D3 was subjected to various processes having different process conditions. The process can be considered in two stages:
- Stage 1 - liquefaction, which includes steps (i) to (iv) of the method of the present invention. In each test run, the following steps were carried out:
   o The crude glycerol and D3 feedstock were mixed and added to the reactor;
   o The temperature was increased to 130°C;
   ∘ 98% sulfuric acid was added in a specified amount (given as catalyst loading in Table 2);
   ∘ The mixture of feedstock and catalyst were held at 130°C with continuous stirring at atmospheric pressure, and under air, for a given reaction time;
   ∘ A required amount of NaOH pellets was added (from 1 to 3 wt.%), and then the neutralized mixture was heated to 170 °C, with the heating from 130 to 170 °C taking 10-15 minutes.
- Stage 2 - further reaction after neutralization, and subsequent dehydration. For each test run, after addition of the NaOH, and reaching 170°C, the contents are held at 170°C for the contents to further react and water and other low boiling compounds to boil off, for a length of time indicated below as "duration of neutralization and dehydration". When the duration of neutralisation and dehydration is 0 mins, it means that as soon as the mixture reaches 170°C, the reaction is stopped. During the neutralization and dehydration stage, the contents of the reactor can be held under an inert atmosphere of N₂, or simply be under normal atmospheric conditions.

The reaction conditions were varied in accordance with Table 2 below.

**Table 2**

| **Test run** | **Reaction time (i.e. duration after adding catalyst and held at elevated temperature)** | **Catalyst loading** | **Duration of neutralisation and dehydration** | **Inert atmosphere during neutralisation and dehydration?** | **Glycerol- or glyceride-containing composition used** |
|---|---|---|---|---|---|
| D3_NoBase | 60 mins | 3 wt.% | 0 minutes | Yes | Crude glycerol with low ash (2.36 wt.% ash) |
| D3_2% | 60 mins | 2 wt.% | 45 mins | Yes | Crude glycerol with low ash |
| D3_90Base | 60 mins | 3 wt.% | 90 mins | Yes | Crude glycerol with low ash |
| D3_4% | 60 mins | 4 wt.% | 45 mins | Yes | Crude glycerol with low ash |
| D3_RCG | 60 mins | 3 wt.% | 45 mins | Yes | Crude glycerol with high ash content (9.5 wt.% ash) |
| D3_Acid120 | 120 mins | 3 wt.% | 45 mins | Yes | Crude glycerol with low ash |
| D3_Acid180 | 180 mins | 3 wt.% | 45 mins | Yes | Crude glycerol with low ash |
| D3_NoN2 | 60 mins | 3 wt.% | 45 mins | No | Crude glycerol with low ash |
| D3_Ultimate | 60 mins | 2 wt.% | 0 mins (i.e no neutralisation or dehydration) | No | Crude glycerol with low ash |

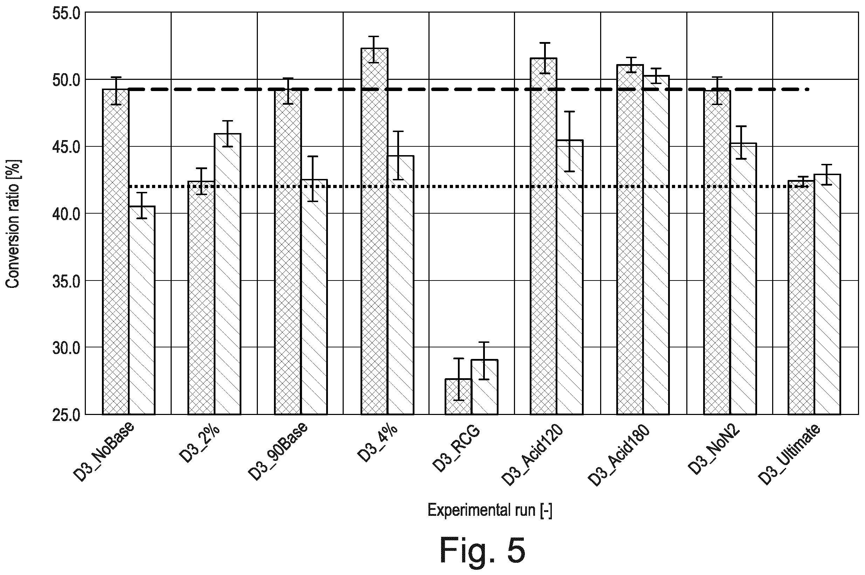

The conversion ratios after stage 1 were measured and are shown in the solid bars of the graph of Figure 5. The conversion ratios after stage 2 (if such a stage 2 was present) were measured, and are displayed in the striped bars in the graph of Figure 5.

The data presented in Figure 5 highlights that the process efficacy is highly dependent on specific process parameters. In cases where stage 2 yields a lower conversion ratio (i.e. D3_NoBase, D3_90Base, D3_4%, D3_Acid180, and D3_NoN2), conditions for poly- and recondensation reactions were favourable. In particular, the reaction equilibrium shifts towards recondensation (formation of higher molecular weight compounds and complexes, which are often solids and/or compounds poorly soluble in glycerol) instead of liquefaction when the concentration of polyols increases in the medium. This can be remediated, for example, by continuously removing biopolyols, and generally is not an issue in plug-flow systems.

On the other hand, in D3_2%, D3_RCG, and D3_Ultimate, for example, the second stage was advantageous in achieving a further increase in extent of liquefaction, suggesting that chemical bond breaking, rather than formation or reconstitution, were favoured.

The biomass conversion ratio (CR) was determined by mixing 2-3 g of biopolyol twice with 50 ml of ethanol, and once with 50 ml of distilled water to dissolve the ethanol precipitates. In between each mixing, the samples were centrifuged and the supernatants discarded. After mixing with water, the mixtures were filtered under vacuum and dried at 105°C. Then the ratios for the acid and base catalysed steps, respectively, were calculated using the following equations: ${CR}_{acid} = 100 - \frac{{m}_{r} ∗ {m}_{tbp}}{{m}_{s} ∗ {m}_{feed}} ∗ 100 %$ and ${CR}_{base} = 100 - \frac{{m}_{r} ∗ {m}_{tbp}}{{m}_{s} ∗ \left({m}_{feed}-{m}_{cfeed}\right)} ∗ 100 %$

Where mᵣ = mass of dry residue, m_{tbp} = total mass of produced biopolyols, mₛ = mass of taken biopolyol sample, m_{feed} = mass of used feedstock, m_{cfeed} = mass of used feedstock corrected for the intermediate sampling procedure.

Although a higher conversion ratio is advantageous, there is a balance between the conversion ratio and the product quality. The starting feedstock of crude glycerol and digested slurry sludge is a low quality feedstock, and it is desirable to provide a process for producing a high quality biopolyol product mixture, whilst retaining a decent conversion ratio.

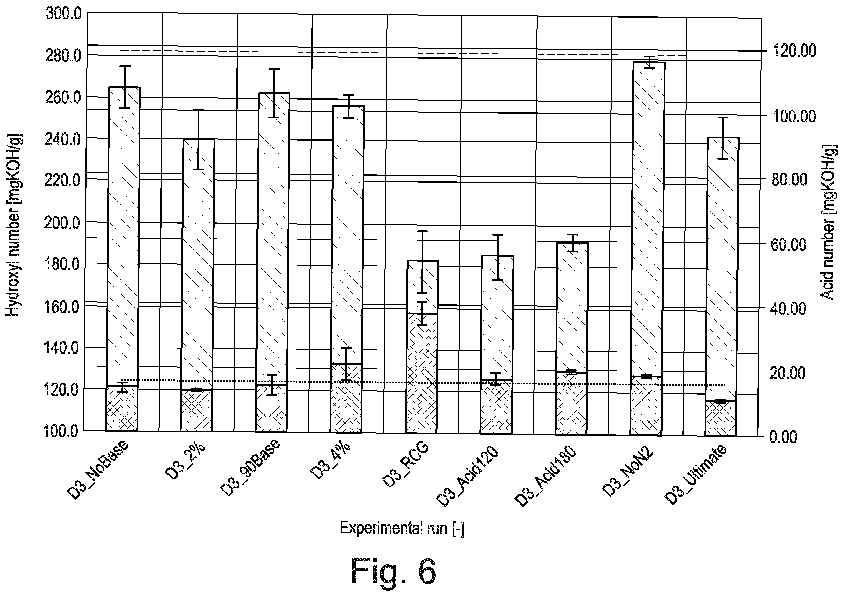

In addition, the hydroxyl number and acid number, in mgKOH/g were measured for each resulting sample (after stages 1 and 2), and the results are displayed in Figure 6. The solid bars show the results for the acid number, and the striped bars show the hydroxyl number.

The data in Figure 6 indicates the quality of the resultant biopolyol product. With regard to the acid number (solid bars), a lower value of said acid number is an indicator of a better biopolyol product quality. The runs with solid bars underneath the dotted line in Figure 6 have a desired low acid number, and include D3_NoBase, D3_2%, D3_90Base, and D3_Ultimate.

In addition, a higher hydroxyl number is desired, indicating a better bio-polyol product quality. As shown in Figure 6, the hydroxyl number is satisfactorily high for all runs except D3_RCG, D3_Acid120, and D3_Acid180.

Thus, when searching for the balance of conversion ratio (yield) with the product quality (acid number and hydroxyl number), D3_ultimate unexpectedly showed the best balance of low acid number, high hydroxyl number, and good conversion ratio. This was unexpected given that the atmosphere was not inert, and the catalyst loading was lower (2 wt.%). In addition, it was surprisingly found that a good conversion ratio and product quality was achieved when no second stage was present (i.e. 0 mins of holding the neutral mixture at 170°C), thereby resulting in a process that takes less time, and is less energy demanding, whilst obtaining a high quality biopolyol product at a good conversion ratio.

### Example 4

A further example was performed, where various feedstocks as indicated for example 1 (D3, H3 and S3) were used in two different processes, one which is the baseline, and one which is denoted "ultimate". The baseline (comparative) process includes the following process conditions:
- 98% sulfuric acid catalyst loading of 3 wt.%
- Heating temperature (during the reaction period after adding the acid catalyst) of 130 °C
- Reaction time (i.e. time held at elevated temperature after addition of acid catalyst) of 60 minutes.
- Pressure and atmosphere before neutralizing agent added: atmospheric pressure under air.
- Neutralizing agent of NaOH pellets, with a loading of approximately 2.7 wt.%, based on the intermediary bio-polyol mixture, (i.e. added after 60 minutes reaction time).
- Neutralization (and dehydration) time (i.e. time held at 170°C after neutralizing agent added and temperature increased from 130 to 170°C): 45 mins
- Average heating rate of 1.5-3.1 °C/min for increasing the temperature from ambient to 130, and from 130 to 170°C.
- An inert atmosphere during neutralization and dehydration step. The inert atmosphere was added immediately preceding addition of the neutralizing agent.

The "ultimate" (inventive) process includes the following process conditions:
- 98% sulfuric acid catalyst loading 2 wt.%.
- Heating temperature 130 °C.
- Reaction time (i.e. time held at elevated temperature after addition of acid catalyst) of 60 minutes.
- Pressure and atmosphere before neutralizing agent added: atmospheric pressure under air.
- Neutralizing agent NaOH pellets, with a loading of approximately 1.6 wt.% by weight of the intermediary biopolyol product, and then the temperature was increased from 130 to 170°C.
- Average heating rate of 1.5-3.1 °C/min for increase in temperature from 130 to 170°C.
- Neutralization (and dehydration) time of 0 mins (after 170°C reached).
- An inert atmosphere during neutralization and dehydration step was not used - it was carried out under air.

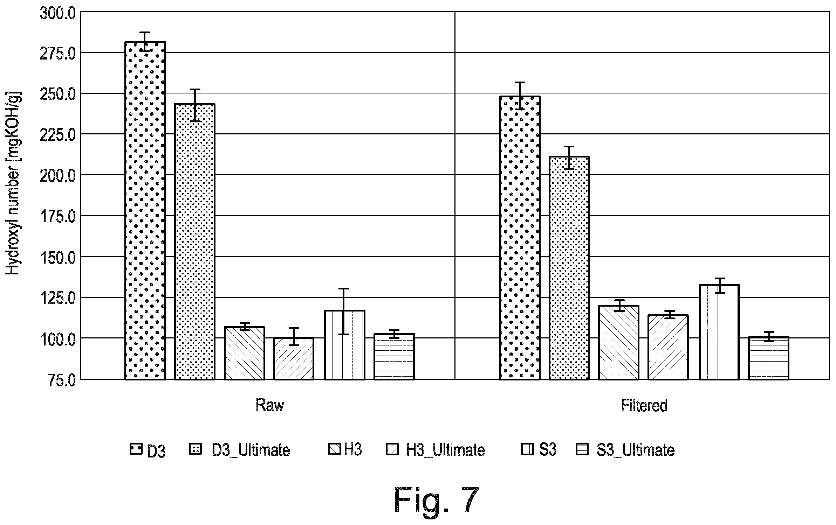

The resulting sample was analysed for hydroxyl number both immediately after the process (Raw), and then again after a filtration step (Filtered). The filtration step includes ethanol mediated vacuum-assisted filtration with solvent removal via rotary evaporation. The results are shown in Figure 7.

The data shows that improved parameters may be observed when comparing filtered biopolyol vs. biopolyol suspension (i.e. as is the case with HSH and SBP). Partially liquefied biomass contains hydroxyl groups on the surface of the remnant structures. Depending on the relative density of these groups, the presence of the solid residues might increase or decrease the overall hydroxyl number of the product. It is clear from Figure 7 that filtration could be a viable technique to slightly increase the overall quality of the final HSH and SBP products, likely because the residues contain low amounts of surface/free/accessible OH groups. For D3, and D3 ultimate, the raw product is already of a good quality, and filtration might not be required, depending on the desired end use.

Although the D3 ultimate run has a slightly lower hydroxyl number, the product quality is still sufficiently high that the benefits of the D3 ultimate process (i.e. lower catalyst loading, no second stage required, no inert atmosphere required) outweigh the slight decrease in product quality. The inventors did not expect that lowering the catalyst amount, removing the second stage, and performing the neutralization under air would be able to achieve a product quality and conversion ratio that are comparable to those produced with a higher catalyst loading, an inert atmosphere, and a stage 2 step present.

### Example 5

A further test was performed to assess the relative properties of the "standard" process against the "ultimate" process, and the resulting foam properties of a polyurethane foam produced by reaction of the biopolyol mixture with a diisocyanate.

The D3 feedstock underwent both the baseline process and the ultimate process, as described above with respect to Example 4. The resulting mixture of each process was taken in its raw (unfiltered) form.

The conversion ratio of the process, and the hydroxyl and acid numbers of the resulting bipolyol product mixture were measured. The biopolyol product mixtures were then reacted with methylene diphenyl diisocyanate (MDI) to form the polyurethane foam according to the following process:
- The biopolyol product mixture, catalyst and surfactant (Kosmos 29 and Tegostab B 8465 by Evonik), and blowing agent (water) were premixed vigorously.
- Pre-weighed MDI (pMDI, Desmodur 44 V 70L by Covestro) was added and the mixture was stirred vigorously with a high-speed stirrer.
- After growing, the foam samples were allowed to cure overnight prior to demolding.
- The foams were then cut into specimens of desired dimensions and conditioned for at least 1 week before testing.

The biopolyol product mixtures were also reacted to form adhesives, and the resulting adhesive tensile strength was measured. The polyurethane composite adhesives were produced using the biopolyol product mixture and commercially available 1,5-pentamethylene diisocyanate (Desmodur eco N7300 by Covestro), with a renewable carbon content of 71%. Dibutyltin dilaurate (Sigma Aldrich) and FoamStop Neutral (Kärcher) were employed as the hardening catalyst and foaming inhibitor, respectively.

The polyurethane foams were tested under ISO172.5 for foam properties, and the polyurethane adhesives were tested under ISO135 for adhesive tensile strength. The results are displayed in Table 3 below.

**Table 3**

| **Processing conditions** | **Conversion ratio (%)** | **Hydroxyl no. (mgKOH/g)** | **Acid no. prior to neutralization (mgKOH/g)** | **Foam compressive strength and apparent density @ISO172.5** | **Adhesive tensile strength @ISO135** |
|---|---|---|---|---|---|
| Baseline | 42.0 ±0.6 | 282 ±6 | 16.3 ±0.7 | 110.7 kPa @ 80.1 kg/m³ | 5.5 MPa |
| Ultimate | 42.8 ±0.7 | 243 ±9 | 10.6 ±0.2 | 60.5 kPa @ 59.9 kg/m³ | 7.0 MPa |

The conditions of the ultimate process were unexpectedly found to have advantages over the baseline process. The results show that the ultimate process conditions yielded a better biopolyol product mixture, because it was able to form strong foams at lower densities, and stronger adhesives, which is largely attributed to an optimal hydroxyl number. The ultimate process required less neutralisation and dehydration demands. This is attributed to a lower intermediary acid number, resulting in less NaOH needed to neutralise the mixture, and less time required to evacuate the formed water, while not compromising the final conversion ratio.

Unless specified to the contrary, percentages herein are by weight.

Although preferred embodiments of the invention have been described herein in detail, it will be understood by those skilled in the art that variations may be made thereto without departing from the scope of the invention or of the appended claims.

## Claims

1. A method for the production of one or more biopolyols from a feedstock, the method comprising:
(i) providing a feedstock comprising waste biomass and a glycerol- or glyceride-containing composition;
(ii) feeding the feedstock to a reactor;
(iii) heating the feedstock in the reactor, together with an acid catalyst, to a first elevated temperature, to form an acid-treated mixture;
(iv) adding a neutralizing agent to the acid-treated mixture to form a neutralising-agent-treated mixture having a pH of at least 6;
(v) heating the neutralising-agent-treated-mixture to a second elevated temperature of at least 130ºC, preferably at least 160 ºC, and most preferably about 170ºC, to form a further mixture;
(vi) optionally, filtering and/or dehydrating the further mixture obtained from step (v);
wherein the method comprises extracting volatiles from the reactor during steps (iii) and/or (iv) and/or (v), and
wherein the acid catalyst is mixed with the feedstock:
a. before heating of the feedstock, and/or
b. during heating of the feedstock, and/or
c. after heating of the feedstock;
and wherein:
(A) in step (v) the further mixture is held at the second elevated temperature for less than 40 minutes, and optionally wherein
(B) in step (v) the further mixture is maintained under air, and/or
(C) in step (iv) the neutralising-agent-treated mixture has a pH of from 6 to about 7;
wherein the reactor is operated:
(a) under air, and/or
(b) at partial vacuum of from 10 to 100 kPa.

2. The method of claim 1, wherein the one or more biopolyols have a moisture content of less than 3wt.%; and/or a hydroxyl number of at least 75 mgKOH/g, and/or an acid number of less than 30 mgkOH/g, and/or a moderate dynamic viscosity of up to 20 Pa.s at 25°C; and/or wherein the reactor is a continuously-stirred tank reactor or a continuous plug flow reactor; wherein the moisture content of the one or more biopolyols is measured according to DIN 51777;
wherein the hydroxyl number of the one or more biopolyols is measured according to DIN 53240;
wherein the acid number of the one or more biopolyols is measured according to DIN 53402; and wherein the moderate dynamic viscosity of the one or more biopolyols is measured according to DIN 53015.

3. The method of any preceding claim, wherein the waste biomass comprises:
(a) sewage, preferably with a dry matter content of at least 10 wt.%; and/or
(b) spent mushroom substrate, preferably with a dry matter content of at least 50 wt.%; and/or
(c) municipal organic waste, preferably comprising food waste and/or paper and/or cardboard and/or gardening waste and/or farming waste and/or macroalgal waste, and wherein the municipal organic waste preferably has a dry matter content of at least 25 wt.%.

4. The method of any preceding claim, wherein the glycerol- or glyceride-containing composition comprises glycerol, and/or vegetable oil, preferably used/waste cooking oil, preferably wherein the glycerol- or glyceride-containing composition comprises, or consists essentially of, or consists of, glycerol, most preferably crude glycerol.

5. The method of any preceding claim, wherein:
(a) the feedstock is pre-mixed before being sent into the reactor in step (ii); and/or
(b) step (iii) includes mixing the feedstock whilst heating; and/or
(c) step (iii) includes heating to a first elevated temperature of from 110 to 170 °C, preferably from 110 to 155, more preferably from 120 to 140 °C, most preferably about 130°C, and wherein once the catalyst-containing mixture has reached the first elevated temperature, the catalyst-containing mixture is held at the first elevated temperature for a period of from 20 mins to 3 hours, more preferably from 30 mins to 2 hours, and most preferably about 60 mins.

6. The method of any preceding claim, wherein as soon as the second elevated temperature is reached, the mixture is held at the second elevated temperature for a period of from 0 to 30 mins, and most preferably 0 mins.

7. The method of any preceding claim, wherein the reactor is operated: (b) at partial vacuum of from 30 to 80 kPa, and preferably from 40 to 60 kPa.

8. The method of any preceding claim, wherein as soon as the second elevated temperature is reached, the mixture is held at the second elevated temperature for a period of from 0 to 15 mins, and wherein the reactor is operated under air, and wherein step (iii) includes adding the catalyst to the mixture in an amount of about 2 wt.%, by dry weight of the initial glycerol- or glyceride- containing composition, and wherein step (iv) includes adding the neutralizing agent in an amount of from 1 to 2.5 wt.%, by weight of the catalyst-containing mixture present at the end of step (iii) to neutralize the mixture.

9. The method of any preceding claim, wherein the acid catalyst is a strong acid with a pKa greater than 1.74, and more preferably wherein the acid catalyst comprises sulphuric acid and/or phosphoric acid and/or hydrochloric acid and/or nitric acid.

10. The method of any preceding claim, wherein the acid catalyst is added to the mixture in an amount of from 0.5 to 5 wt.%, by dry weight of the initial glycerol- or glyceride- containing composition, preferably in an amount of from 1.5 to 2.5 wt.%, and more preferably about 2 wt.%.

11. The method of any preceding claim, wherein step (iv) includes adding the neutralizing agent in an amount of from 0.2 to 5 wt.%, preferably from 0.5 to 3 wt.%, and more preferably from 1 to 2.5 wt.%, by weight of the acid-treated mixture present at the end of step (iii).

12. The method of any preceding claim, wherein the liquid to solid ratio of the feedstock is from 15:1 to 3:1.

13. The method of any preceding claim, wherein the combination of steps (iii) to (v) have a duration of from 30 minutes to 6 hours.

14. The method of any preceding claim, wherein:
(a) the catalyst is added to the feedstock before heating, and the mixture of catalyst and feedstock is heated to a temperature of from 110 to 170°C, and then held at this temperature for a period of from 15 minutes to 5 hours; or
(b) the catalyst is added to the feedstock during heating, and the mixture of catalyst and feedstock is heated to a temperature of from 110 to 170°C, and then held at this temperature for a period of from 15 minutes to 5 hours; or
(c) the catalyst is added to the feedstock after heating, and the mixture of catalyst and feedstock is held at a temperature of from 110 to 170°C for a period of from 15 minutes to 5 hours.

15. The method of any preceding claim, wherein the pressure of the reactor is maintained at from 10 to 100 kPa, preferably 30 to 80 kPa, and more preferably at about 50 kPa.

## Patentansprüche

1. Verfahren zur Herstellung eines oder mehrerer Biopolyole aus einem Ausgangsmaterial, wobei das Verfahren Folgendes umfasst:
(i) Bereitstellen eines Ausgangsmaterials, das Abfallbiomasse und eine glycerin- oder glyceridhaltige Zusammensetzung umfasst;
(ii) Zuführen des Ausgangsmaterials zu einem Reaktor;
(iii) Erhitzen des Ausgangsmaterials im Reaktor zusammen mit einem Säurekatalysator auf eine erste erhöhte Temperatur, um eine säurebehandelte Mischung zu bilden;
(iv) Zugeben eines Neutralisationsmittels zu der säurebehandelten Mischung, um eine mit Neutralisationsmittel behandelte Mischung mit einem pH-Wert von mindestens 6 zu bilden;
(v) Erhitzen der mit Neutralisationsmittel behandelten Mischung auf eine zweite erhöhte Temperatur von mindestens 130 °C, vorzugsweise mindestens 160 °C und besonders bevorzugt etwa 170 °C, um eine weitere Mischung zu bilden;
(vi) optional, Filtern und/oder Dehydrieren der weiteren, aus Schritt (v) erhaltenen Mischung;
wobei das Verfahren Extrahieren flüchtiger Bestandteile aus dem Reaktor während der Schritte (iii) und/oder (iv) und/oder (v) umfasst, und
wobei der Säurekatalysator mit dem Ausgangsmaterial vermischt wird:
a. vor dem Erhitzen des Ausgangsmaterials, und/oder
b. während des Erhitzens des Ausgangsmaterials, und/oder
c. nach dem Erhitzen des Ausgangsmaterials;
und wobei:
(A) in Schritt (v) die weitere Mischung weniger als 40 Minuten lang bei der zweiten erhöhten Temperatur gehalten wird, und wobei optional:
(B) in Schritt (v) die weitere Mischung unter Luftzufuhr gehalten wird, und/oder
(C) in Schritt (iv) die mit dem Neutralisationsmittel behandelte Mischung einen pH-Wert von 6 bis etwa 7 aufweist;
wobei der Reaktor betrieben wird:
(a) unter Luft, und/oder
(b) bei einem Teilvakuum von 10 bis 100 kPa.

2. Verfahren nach Anspruch 1, wobei das eine oder die mehreren Biopolyole einen Feuchtigkeitsgehalt von weniger als 3 Gew.-%; und/oder eine Hydroxylzahl von mindestens 75 mgKOH/g, und/oder eine Säurezahl von weniger als 30 mgkOH/g, und/oder eine moderate dynamische Viskosität von bis zu 20 Pa·s bei 25 °C aufweisen; und/oder
wobei es sich bei dem Reaktor um einen kontinuierlich gerührten Tankreaktor oder einen kontinuierlich betriebenen Strömungsrohrreaktor handelt;
wobei der Feuchtigkeitsgehalt des einen oder der mehreren Biopolyole nach DIN 51777 gemessen wird;
wobei die Hydroxylzahl des einen oder der mehreren Biopolyole nach DIN 53240 gemessen wird;
wobei die Säurezahl des einen oder der mehreren Biopolyole nach DIN 53402 gemessen wird; und wobei die moderate dynamische Viskosität des einen oder der mehreren Biopolyole nach DIN 53015 gemessen wird.

3. Verfahren nach einem vorstehenden Anspruch, wobei die Abfallbiomasse Folgendes umfasst:
(a) Abwasser, vorzugsweise mit einem Trockenmassegehalt von mindestens 10 Gew.-%; und/oder
(b) verbrauchtes Pilzsubstrat, vorzugsweise mit einem Trockenmassegehalt von mindestens 50 Gew.-%; und/oder
(c) kommunale organische Abfälle, die vorzugsweise Lebensmittelabfälle und/oder Papier und/oder Karton und/oder Gartenabfälle und/oder landwirtschaftliche Abfälle und/oder Makroalgenabfälle umfassen, wobei die kommunalen organischen Abfälle vorzugsweise einen Trockenmassegehalt von mindestens 25 Gew.-% aufweisen.

4. Verfahren nach einem vorstehenden Anspruch, wobei die glycerin- oder glyceridhaltige Zusammensetzung Glycerin und/oder Pflanzenöl, vorzugsweise gebrauchtes/Abfallspeiseöl umfasst, wobei die glycerin- oder glyceridhaltige Zusammensetzung vorzugsweise Glycerin, besonders bevorzugt Rohglycerin, umfasst oder im Wesentlichen daraus besteht oder daraus besteht.

5. Verfahren nach einem vorstehenden Anspruch, wobei:
(a) das Ausgangsmaterial vorgemischt wird, bevor es in Schritt (ii) in den Reaktor gegeben wird; und/oder
(b) Schritt (iii) Mischen des Ausgangsmaterials während des Erhitzens einschließt; und/oder
(c) Schritt (iii) Erhitzen auf eine erste erhöhte Temperatur von 110 bis 170 °C, vorzugsweise von 110 bis 155, bevorzugter von 120 bis 140 °C, besonders bevorzugt etwa 130 °C einschließt, und wobei die katalysatorhaltige Mischung, sobald sie die erste erhöhte Temperatur erreicht hat, für einen Zeitraum von 20 Minuten bis 3 Stunden, bevorzugter von 30 Minuten bis 2 Stunden und besonders bevorzugt etwa 60 Minuten bei der ersten erhöhten Temperatur gehalten wird.

6. Verfahren nach einem vorstehenden Anspruch, wobei die Mischung, sobald die zweite erhöhte Temperatur erreicht ist, für einen Zeitraum von 0 bis 30 Minuten, besonders bevorzugt 0 Minuten, bei der zweiten erhöhten Temperatur gehalten wird.

7. Verfahren nach einem vorstehenden Anspruch, wobei der Reaktor betrieben wird:
(b) bei einem Teilvakuum von 30 bis 80 kPa, und vorzugsweise von 40 bis 60 kPa.

8. Verfahren nach einem vorstehenden Anspruch, wobei die Mischung, sobald die zweite erhöhte Temperatur erreicht ist, für einen Zeitraum von 0 bis 15 Minuten bei der zweiten erhöhten Temperatur gehalten wird, und wobei der Reaktor unter Luft betrieben wird und wobei Schritt (iii) Zugeben des Katalysators zur Mischung in einer Menge von etwa 2 Gew.-%, bezogen auf das Trockengewicht der ursprünglichen glycerin- oder glyceridhaltigen Zusammensetzung, einschließt, und wobei Schritt (iv) Zugeben des Neutralisationsmittels in einer Menge von 1 bis 2,5 Gew.-%, nach Gewicht der am Ende von Schritt (iii) vorhandenen katalysatorhaltigen Mischung zur Neutralisierung der Mischung einschließt.

9. Verfahren nach einem vorstehenden Anspruch, wobei der Säurekatalysator eine starke Säure mit einem pKa-Wert größer als 1,74 ist, und besonders bevorzugt, wobei der Säurekatalysator Schwefelsäure und/oder Phosphorsäure und/oder Salzsäure und/oder Salpetersäure umfasst.

10. Verfahren nach einem vorstehenden Anspruch, wobei der Säurekatalysator der Mischung in einer Menge von 0,5 bis 5 Gew.-%, bezogen auf das Trockengewicht der ursprünglichen glycerin- oder glyceridhaltigen Zusammensetzung, vorzugsweise in einer Menge von 1,5 bis 2,5 Gew.-%, und bevorzugter etwa 2 Gew.-% zugegeben wird.

11. Verfahren nach einem vorstehenden Anspruch, wobei Schritt (iv) Zugeben des Neutralisationsmittels in einer Menge von 0,2 bis 5 Gew.-%, vorzugsweise von 0,5 bis 3 Gew.-%, und bevorzugter von 1 bis 2,5 Gew.-%, nach Gewicht der säurebehandelten Mischung, die am Ende von Schritt (iii) vorliegt, einschließt.

12. Verfahren nach einem vorstehenden Anspruch, wobei das Verhältnis von Flüssigkeit zu Feststoff im Ausgangsmaterial von 15:1 bis 3:1 beträgt.

13. Verfahren nach einem vorstehenden Anspruch, wobei die Kombination der Schritte (iii) bis (v) eine Dauer von 30 Minuten bis 6 Stunden aufweist.

14. Verfahren nach einem vorstehenden Anspruch, wobei:
(a) der Katalysator dem Ausgangsmaterial vor dem Erhitzen zugegeben wird, und die Mischung aus Katalysator und Ausgangsmaterial auf eine Temperatur von 110 bis 170 °C erhitzt wird und dann für einen Zeitraum von 15 Minuten bis 5 Stunden bei dieser Temperatur gehalten wird; oder
(b) der Katalysator während des Erhitzens dem Ausgangsmaterial zugegeben wird, und die Mischung aus Katalysator und Ausgangsmaterial auf eine Temperatur von 110 bis 170 °C erhitzt wird und dann für einen Zeitraum von 15 Minuten bis 5 Stunden bei dieser Temperatur gehalten wird; oder
(c) der Katalysator nach dem Erhitzen dem Ausgangsmaterial zugegeben wird, und die Mischung aus Katalysator und Ausgangsmaterial für einen Zeitraum von 15 Minuten bis 5 Stunden bei einer Temperatur von 110 bis 170 °C gehalten wird.

15. Verfahren nach einem vorstehenden Anspruch, wobei der Druck im Reaktor auf 10 bis 100 kPa, vorzugsweise 30 bis 80 kPa und bevorzugter auf etwa 50 kPa gehalten wird.

## Revendications

1. Procédé destiné à la production d'un ou plusieurs biopolyols à partir d'une matière première, le procédé comprenant :
(i) la fourniture d'une matière première comprenant de la biomasse résiduelle et une composition contenant du glycérol ou du glycéride ;
(ii) l'alimentation d'un réacteur en matière première ;
(iii) le chauffage de la matière première dans le réacteur, avec un catalyseur acide, à une première température élevée, pour former un mélange traité à l'acide ;
(iv) l'ajout d'un agent neutralisant au mélange traité à l'acide pour former un mélange traité à l'agent neutralisant présentant un pH d'au moins 6 ;
(v) le chauffage du mélange traité à l'agent neutralisant à une seconde température élevée d'au moins 130 °C, de préférence d'au moins 160 °C, et le plus préférentiellement d'environ 170 °C, pour former un autre mélange ;
(vi) éventuellement, le filtrage et/ou la déshydratation de l'autre mélange obtenu à l'étape (v) ;
dans lequel le procédé comprend l'extraction de composés volatils du réacteur au cours des étapes (iii) et/ou (iv) et/ou (v), et
dans lequel le catalyseur acide est mélangé à la matière première :
a. avant le chauffage de la matière première, et/ou
b. pendant le chauffage de la matière première, et/ou
c. après le chauffage de la matière première ;
et dans lequel :
(A) à l'étape (v), l'autre mélange est maintenu à la seconde température élevée pendant moins de 40 minutes, et éventuellement dans lequel
(B) à l'étape (v), l'autre mélange est conservé sous air, et/ou
(C) à l'étape (iv), le mélange traité à l'agent neutralisant présente un pH compris de 6 à environ 7 ;
dans lequel le réacteur fonctionne :
(a) sous air, et/ou
(b) dans un vide partiel de 10 à 100 kPa.

2. Procédé selon la revendication 1, dans lequel les un ou plusieurs biopolyols présentent une teneur en humidité inférieure à 3 % en poids ; et/ou un indice hydroxyle d'au moins 75 mgKOH/g, et/ou un indice acide inférieur à 30 mgkOH/g, et/ou une viscosité dynamique modérée pouvant atteindre 20 Pa·s à 25 °C ; et/ou
dans lequel le réacteur est un réacteur à cuve agitée en continu ou un réacteur à flux piston continu ;
dans lequel la teneur en humidité des un ou plusieurs biopolyols est mesurée selon la norme DIN 51777 ;
dans lequel l'indice hydroxyle des un ou plusieurs biopolyols est mesuré selon la norme DIN 53240 ;
dans lequel l'indice acide des un ou plusieurs biopolyols est mesuré selon la norme DIN 53402 ; et dans lequel la viscosité dynamique modérée des un ou plusieurs biopolyols est mesurée selon la norme DIN 53015.

3. Procédé selon une quelconque revendication précédente, dans lequel la biomasse résiduelle comprend :
(a) des eaux usées, de préférence avec une teneur en matière sèche d'au moins 10 % en poids ; et/ou
b) un substrat de champignon épuisé, de préférence avec une teneur en matière sèche d'au moins 50 % en poids ; et/ou
(c) des déchets organiques municipaux, comprenant de préférence des déchets alimentaires et/ou du papier et/ou du carton et/ou des déchets verts et/ou des déchets issus de l'agriculture et/ou des déchets de macroalgues, et dans lequel les déchets organiques municipaux présentent de préférence une teneur en matière sèche d'au moins 25 % en poids.

4. Procédé selon une quelconque revendication précédente, dans lequel la composition contenant du glycérol ou du glycéride comprend du glycérol, et/ou une huile végétale, de préférence une huile de cuisson usagée/résiduelle, de préférence dans lequel la composition contenant du glycérol ou du glycéride comprend, ou consiste essentiellement en, ou consiste en, du glycérol, le plus préférentiellement du glycérol brut.

5. Procédé selon une quelconque revendication précédente, dans lequel :
(a) la matière première est prémélangée avant d'être envoyée dans le réacteur à l'étape (ii) ; et/ou
(b) l'étape (iii) inclut le mélange de la matière première pendant le chauffage ; et/ou
(c) l'étape (iii) inclut le chauffage à une première température élevée de 110 à 170 °C, de préférence de 110 à 155 °C, plus préférentiellement de 120 à 140 °C, le plus préférentiellement d'environ 130 °C, et dans lequel une fois que le mélange contenant le catalyseur a atteint la première température élevée, le mélange contenant le catalyseur est maintenu à la première température élevée pendant une période de 20 minutes à 3 heures, plus préférentiellement de 30 minutes à 2 heures, et le plus préférentiellement d'environ 60 minutes.

6. Procédé selon une quelconque revendication précédente, dans lequel, dès que la seconde température élevée est atteinte, le mélange est maintenu à la seconde température élevée pendant une période de 0 à 30 minutes, et le plus préférentiellement 0 minute.

7. Procédé selon une quelconque revendication précédente, dans lequel le réacteur fonctionne :
(b) dans un vide partiel de 30 à 80 kPa, et de préférence de 40 à 60 kPa.

8. Procédé selon une quelconque revendication précédente, dans lequel, dès que la seconde température élevée est atteinte, le mélange est maintenu à la seconde température élevée pendant une durée de 0 à 15 minutes, et dans lequel le réacteur fonctionne sous air, et dans lequel l'étape (iii) inclut l'ajout du catalyseur au mélange en une quantité d'environ 2 % en poids, en poids sec de la composition initiale contenant du glycérol ou du glycéride, et dans lequel l'étape (iv) inclut l'ajout de l'agent neutralisant en une quantité de 1 à 2,5 % en poids, en poids du mélange contenant le catalyseur présent à la fin de l'étape (iii) pour neutraliser le mélange.

9. Procédé selon une quelconque revendication précédente, dans lequel le catalyseur acide est un acide fort dont le pKa est supérieur à 1,74, et plus préférentiellement dans lequel le catalyseur acide comprend de l'acide sulfurique et/ou de l'acide phosphorique et/ou de l'acide chlorhydrique et/ou de l'acide nitrique.

10. Procédé selon une quelconque revendication précédente, dans lequel le catalyseur acide est ajouté au mélange en une quantité de 0,5 à 5 % en poids, en poids sec de la composition initiale contenant du glycérol ou du glycéride, de préférence en une quantité de 1,5 à 2,5 % en poids, et plus préférentiellement d'environ 2 % en poids.

11. Procédé selon une quelconque revendication précédente, dans lequel l'étape (iv) comprend l'ajout de l'agent neutralisant en une quantité de 0,2 à 5 % en poids, de préférence de 0,5 à 3 % en poids, et plus préférentiellement de 1 à 2,5 % en poids, en poids du mélange traité à l'acide présent à la fin de l'étape (iii).

12. Procédé selon une quelconque revendication précédente, dans lequel le rapport liquide/solide de la matière première est de 15:1 à 3:1.

13. Procédé selon une quelconque revendication précédente, dans lequel la combinaison des étapes (iii) à (v) présente une durée de 30 minutes à 6 heures.

14. Procédé selon une quelconque revendication précédente, dans lequel :
a) le catalyseur est ajouté à la matière première avant le chauffage, et le mélange de catalyseur et de matière première est chauffé à une température de 110 à 170 °C, puis maintenu à cette température pendant une période de 15 minutes à 5 heures ; ou
b) le catalyseur est ajouté à la matière première pendant le chauffage, et le mélange de catalyseur et de matière première est chauffé à une température de 110 à 170 °C, puis maintenu à cette température pendant une période de 15 minutes à 5 heures ; ou
(c) le catalyseur est ajouté à la matière première après le chauffage, et le mélange de catalyseur et de matière première est maintenu à une température de 110 à 170 °C pendant une période de 15 minutes à 5 heures.

15. Procédé selon une quelconque revendication précédente, dans lequel la pression du réacteur est conservée de 10 à 100 kPa, de préférence de 30 à 80 kPa, et plus préférentiellement à environ 50 kPa.
